# EUROPEAN PATENT APPLICATION

(11) **EP 4 834 248 A1**
(43) Date of publication of application: **04.11.2026**
(21) Application number: 25173815.9
(22) Date of filing: 30.04.2025
(51) Int. Cl.: A61B 17/17

(54) **DRILL GUIDE**

(71) Applicant: PMU Innovations GmbH, 5020 Salzburg (AT)
(72) Inventor: FIERLBECK, Johann, 83435 Bad Reichenhall (DE)
(74) Representative: Vossius & Partner Patentanwälte Rechtsanwälte mbB

(57) **Abstract**

A universal drill guide (10, 100) for drilling and/or positioning an acetabular screw into a left and right hip bone for treatment of acetabular fractures is proposed. The drill guide (10, 100) comprises: a guide plate (12) having a main body (14), a first contact surface (16) formed on the main body (14) and configured for contacting a first region of the hip bone, a through-hole (44) arranged in the first contact surface (16) and extending through the main body (14), the through-hole (44) extending along a through-hole axis (18), the through-hole axis defining a drilling axis for drilling into the hip bone, and an extension body (20) extending from the main body (14) in a direction of the through-hole axis (18), wherein the extension body (20) includes a root section (22) and a tip section (24), the root section (22) being arranged closer to the main body (14) than the tip section (24), the extension body (20) is formed such that the tip section (24) is arranged closer to the through-hole axis (18) than the root section (22), and the tip section (24) forms a second contact surface (26) configured for contacting a second region of the hip bone, the second contact surface (26) being arranged at an angle to the first contact surface (16) and being configured for contacting the left and right hip bone.

## Description

### Field of the Invention

The present invention relates to the field of orthopedic surgery, specifically to the placement of infra-acetabular screws (IAS) and acetabular posterior column screws (APCS) in the treatment of acetabular fractures. The present invention further relates to a universal drill guide (universal drilling template) configured for drilling and/or positioning an IAS or APCS into a left and right hip bone using common anatomical variations of the left and right hip bone.

### Background of the Invention

Acetabular fractures can range from patterns, such as anterior column or posterior column disruptions, to more complex arrangements like T-type and both-column fractures. For many of these injury patterns, especially those addressed through an ilioinguinal approach or requiring posterior column stabilization, precise screw placement is critical for achieving stable fixation and optimal patient outcomes.

In current orthopedic practice, various techniques and/or methods have been developed to address the complexities of placing screws in acetabular fractures.

As an example, freehand techniques remain a common approach due to their straightforward equipment requirements. However, the lack of guiding aids means there is a high risk of misplacement, cortical perforation, and damage to nearby neurovascular structures. These risks increase dramatically in the confined infra-acetabular and posterior column corridors, where even minor deviations in screw angle can lead to complications such as joint penetration or vessel injury.

Another example is computer-assisted navigation. Computer-assisted navigation has emerged to improve the accuracy of screw placement by offering real-time guidance. This approach significantly reduces the risk of cortical breaches and neurovascular damage. Nonetheless, its adoption is limited by high equipment costs, extensive setup time, and the need for specialized training. In many resource-limited settings, navigation systems are simply not available, making them an impractical solution for widespread use.

Another example is to employ individualized drilling templates based on a patient's computed tomography (CT) data. These templates can guide screws with a high degree of precision, reducing the guesswork during surgery. However, this option requires advanced imaging resources and access to 3D printing facilities. The manufacturing process introduces both time and logistical constraints, which can pose significant challenges when urgent surgical intervention is needed. Additionally, if the intra operative achieved reduction is not matching the planed result, , the rigid, predesigned template lacks flexibility for on-the-spot modifications.

However, despite their usefulness and specific advantages, each of the mentioned methods presents its own specific challenges in terms of accuracy, cost, and surgical feasibility.

A further challenge in treating acetabular fractures is that both infra-acetabular screws (IAS) and acetabular posterior column screws (APCS) must be positioned through narrow corridors, placing them in proximity to critical vascular and neural structures. The inherently high complexity of the acetabular anatomy, with its three-dimensional contours, necessitates meticulous control over screw orientation and depth. Even minor deviations can lead to significant complications, including joint penetration, neurovascular damage, and insufficient fixation-problems exacerbated in osteoporotic bone, where screw purchase may be poor.

Although the current array of techniques, ranging from freehand placement to navigation and patient-specific guides, can mitigate some of these challenges, they often introduce further drawbacks. Notably, high costs, prolonged operative times, or impractical lead times for manufacturing reduce the accessibility and efficiency of these solutions.

In addition, the successful stabilization of acetabular fractures, particularly those requiring infra-acetabular screws and acetabular posterior column screws, is hindered by several technical challenges.

While guided approaches, such as computer-assisted navigation or patient-specific drilling guides, can help improve accuracy, they introduce significant drawbacks in terms of cost, time, and operative complexity. These issues become even more pronounced when dealing with the complex anatomy of the pelvis, and, for example, if there is a need to reposition the patient during the operation for another surgical approach.

For example, a lack of intraoperative flexibility arises because pre-manufactured drilling templates cannot accommodate unexpected surgical findings or changes in fracture alignment once surgery has commenced. Such rigidity may force surgeons to abandon templates mid-procedure, increasing both operative time and the risk of inaccuracies.

Another challenge is the time and cost constraints. For example, advanced navigation equipment and patient-specific template manufacturing both demand extensive imaging, planning, and production processes, which may be impractical in urgent scenarios or in settings with limited resources.

Another difficulty relates to consistency and accuracy. Freehand placement demands exceptional surgical skills. Learning to use navigation systems can be complex and time-consuming. Both methods risk deviation from the planned trajectory, especially for surgeons who do not regularly perform these procedures.

Finally, the risk of neurovascular injury looms large because the corridors for IAS and APCS lie close to critical structures, offering limited safe zones for screw placement.

Thus, there is a clear need for a more balanced approach that combines precision, cost-effectiveness, and flexibility in screw placement strategies for the infra-acetabular and posterior column screw.

It is thus an object of the present invention to design a versatile, cost-effective, and user-friendly drill guide (drilling template) that addresses anatomical complexity and mitigates neurovascular hazards without imposing excessive logistical or financial burdens on the surgical workflow.

### Solution to the Problem

This and other objects, which become apparent upon reading the description, are solved by the subject-matter of independent claim 1. Further embodiments and developments are provided in the dependent claims.

According to an aspect of the present invention, a universal drill guide for drilling and/or positioning an acetabular screw, such as an IAS or APCS, into a left and right hip bone for treatment of acetabular fractures is proposed. The wording "left and right hip bone" means that the drill guide is equally configured for being placed on the left hip bone and on the right hip bone, albeit not at the same time. In other words, the same drill guide may be interchangeably used for being placed on the left hip bone and on the right hip bone for treating fractures of the left acetabulum or the right acetabulum, respectively.

Within this disclosure, the hip bone (os coxae) is the large, irregularly shaped bone that forms part of the pelvic girdle. It is composed of three fused bones: ilium (os ilium), ischium (os ischii) and pubis (Os pubis). Os ilium is the largest and most superior portion, forming the broad, flaring part of the hip. It includes landmarks like the iliac crest, anterior superior iliac spine (ASIS), and greater sciatic notch. Os ischii is the posterior-inferior part, contributing to the acetabulum and forming the ischial tuberosity, which supports body weight while sitting. Os pubis is the anterior-inferior portion, meeting its counterpart at the pubic symphysis and forming the obturator foramen with the ischium. These three bones fuse at the acetabulum, the deep socket that articulates with the femoral head to form the hip joint.

The proposed drill guide comprises: a guide plate having a main body, a first contact surface formed on the main body and configured for contacting a first region of the hip bone, a through-hole arranged in or on the first contact surface and extending through the main body, the through-hole extending along a through-hole axis, the through-hole axis defining a drilling axis for drilling into the hip bone, and/or defining a positioning axis for positioning the acetabular screw into the hip bone, and an extension body extending from the main body in a direction of the through-hole axis. By the wording "the extension body extends from the main body in a direction of the through-hole axis" is meant that the extension body has a substantial elongation in the through-hole axis direction. The wording shall not be misinterpreted in a way that the extension body is attached to the main body at a position overlapping with the through-hole axis. In fact, the extension body may be attached to the main body at any appropriate location as long as it extends in a direction of the through-hole axis. The extension body includes a root section and a tip section, the root section being arranged closer to the main body than the tip section. In other words, the root section is connected to the main body and the tip section is connected to the root section. The extension body is formed such that the tip section is arranged closer to the through-hole axis than the root section and such that the tip section forms a second contact surface configured for contacting a second region of the hip bone, wherein the second contact surface is arranged at an angle to the first contact surface and wherein the second contact surface is configured for contacting the left and right hip bone.

The present invention is based on the idea that unlike patient-specific guides which are designed to fit either on the left or right hip bone of a specific patient, the proposed drill guide is designed to fit on both, the left and right hip bone and for a majority of acetabular anatomies and/or patients. The idea is to have a single universal drill guide for the left and right hip bone as opposed to lots of different and patient specific drill guides that either fit to the left or right hip bone. The use of a universal drill guide reduces manufacturing lead times and overall costs. The guide is designed to allow the surgeon to access the entry points for acetabular screw placement as described in the literature. The guide is designed to align with specific landmarks of the hip bone that are common in a majority of anatomies and/or patients. The two contact surfaces of the drill guide enable proper placement of the drill guide on the left and right hip bone at the correct angular orientation with respect to the left and right hip bone. The design of the drill guide allows the surgeon to use the drill guide interchangeably on the left and right hip bone. By avoiding the need for custom, single-use model drill guide, the proposed universal drill guide streamlines preoperative planning and can be readily applied in urgent or standard surgical settings. In addition, the use on both, the left and right side of the body, further increases safety during use.

In a preferred embodiment, in a cartesian coordinate system having an x-axis, a y-axis and a z-axis intersecting each other in a common origin (O), the through-hole axis extends along the z-axis, an xz-plane extends centrally through the extension body, the main body includes a first dimension measured along the x-axis that is larger than a second dimension of the main body measured along the y-axis, and/or the first contact surface includes a first dimension measured along the x-axis that is larger than a second dimension of the first contact surface measured along the y-axis, preferably wherein the first dimension is larger than the second dimension by a factor of four, preferably three, more preferably 2.5, even more preferably 1.3, and the second contact surface includes a first dimension measured along the z-axis that is larger than a second dimension of the second contact surface measured along the y-axis, preferably wherein the first dimension is larger than the second dimension by a factor of four, preferably 3.5.

Preferably, wherein, in a cartesian coordinate system having an x-axis, a y-axis and a z-axis intersecting each other in a common origin (O), the through-hole axis extends along the z-axis, an xz-plane extends centrally through the extension body, and the extension body includes a length measured along the z-axis that is larger than a width of the extension body measured along the y-axis. In other words, a length of the extension body (measured in z-direction) is larger than a width of the extension body (measured in y-direction).

Preferably, the first contact surface is configured for contacting a top region of the left or right hip bone and the second contact surface is configured for contacting a side region of the left or right hip bone.

Preferably, the extension body includes a finger-like shape. The finger-like shape may align with the shape of the quadrilateral surface. The surgeon may use the finger-like shape of the extension body to verify the correct placement and/or intended positioning using his or her finger aligned with the finger-like shape of the extension body.

Preferably, wherein, in a cartesian coordinate system having an x-axis, a y-axis and a z-axis intersecting each other in a common origin (O), the through-hole axis extends along the z-axis, and the extension body includes, at least in the region around the second contact surface, a width measured along the y-axis, wherein the width of the extension body is adapted to a width of a preferred drill corridor in the hip bone, preferably wherein the preferred drill corridor is an infra-acetabular corridor or a posterior column corridor , and/or wherein the width of the extension body is in a range between 2 mm and 9 mm, preferably between 5 mm and 8 mm, more preferably between 6 mm and 6.5 mm, and/or the extension body includes a length measured along the z-axis that is larger than the width measured along the y-axis. The infra-acetabular corridor may be delimited superior by the acetabular notch (part of the hip joint), inferior by the obturator foramen (formed by the ischium and pubis), medially by the quadrilateral surface (thin bony region of the acetabulum), and laterally by the outer cortex of the ischium . The posterior column corridor may be delimited superior by the greater sciatic notch, inferior by the ischial tuberosity, medially by the pelvic brim and quadrilateral surface , and laterally by the outer cortex of the ischium.

Preferably, wherein, in a cartesian coordinate system having an x-axis, a y-axis and a z-axis intersecting each other in a common origin (O), the through-hole axis extends along the z-axis, and the first contact surface, when viewed in an yz-plane, includes a convex shape, the convex shape being configured for contacting a top region of the left and right hip bone, and/or enabling a tilting of the guide plate for abutting on the left and right hip bone, preferably wherein the first contact surface includes beveled or chamfered side portions. By the term "convex shape" is meant an outwardly curved shape as opposed to a flat or planar shape. In other words, when viewed in an yz-plane, the first contact surface has an outwardly (which means pointing outwards and away from the main body) curved shape.

Preferably, the guide plate includes fixation spikes for fixating the guide plate, preferably wherein fixation is such that the guide plate is fixated on the hip bone against unwanted movement, preferably wherein the fixation spikes are arranged perpendicular on the first contact surface. Preferably, the fixation spikes are arranged on the first contact surface only. Fixation spikes ensure initial grip on the bone, effectively preventing unwanted movement. The fixation spikes optimize guide placement and prevent slipping.

Preferably, wherein, in a cartesian coordinate system having an x-axis, a y-axis and a z-axis intersecting each other in a common origin (O), the fixation spikes are arranged symmetrically with respect to an xz-plane. The symmetric arrangement of the fixation spikes improves consistent functionality and haptic feed-back for the surgeon on both the left and right hip bone.

Preferably, the fixation spikes are arranged symmetrically with respect to an yz-plane and/or with respect to the xz-plane.

Preferably, the fixation spikes are arranged such that an acetabular reconstruction plate fits underneath the guide plate without overlapping with the fixation spikes.

Preferably, the fixation spikes are spaced apparat from one another by at least 5mm, more preferably by at least 6 mm, even more preferably by at least 7 mm, and/or by 18 mm or less, more preferably by 16 mm or less, even more preferably by 14 mm or less, and/or in a range between 5 mm to 18 mm, more preferably between 6 mm to 16 mm, even more preferably between 7 mm and 14 mm.

Preferably, the guide plate is shaped to be placed in combination with an acetabular reconstruction plate, and/or includes indentations and/or elevations configured for accommodating the geometrical shape of an acetabular reconstruction plate, preferably wherein the indentations and/or elevations have a height adapted to the thickness of the reconstruction plate. This embodiment allows the surgeon to position both the reconstruction plate and use the drill guide in combination with the reconstruction plates, facilitating the surgical workflow and reducing the risk of misalignment when combining the two fixation methods.

Preferably, the main body and preferably also the extension body is made from a radiolucent material. Within this disclosure a "radiolucent material" is a material that allows transmission of X-rays through it, as opposed to a "radiopaque material" which (substantially) prevents transmission of X-rays through it.

Preferably, the extension body includes a radiographic marker, preferably wherein the radiographic marker is made from radiopaque material, and/or the radiographic marker indicates an alignment of the through-hole axis with respect to a preferred drill corridor, and/or the radiographic marker is positioned in a region of the second contact surface, preferably only in a region of the second contact surface. This preferred embodiment is based on the idea that radiographic markers (such as metallic markers) preferably in combination with a main body and an extension body made of radiolucent material, can help the surgeon verify the intended screw trajectory and/or angulation without obscuring critical anatomy. Radiographic markers may lower the number of repeated fluoroscopic checks and may thus decrease radiation exposure to both patient and surgical staff.

Preferably, wherein, in a cartesian coordinate system having an x-axis, a y-axis and a z-axis intersecting each other in a common origin (O), the through-hole axis extends along the z-axis, the xz-plane may extend centrally through the extension body, and the radiographic marker is a wire, preferably a metallic wire, preferably wherein the wire extends along a wire axis, the wire axis overlapping with the through-hole axis when viewed in a direction along the x-axis. This preferred embodiment is based on the idea that the radiographic wire may be positioned such that, when viewed in a direction along the x-axis, the wire axis and the through-hole axis overlap each other. As the wire axis and the through-hole axis overlap each other when viewed in a direction along the x-axis, the surgeon may verify the correct alignment of the intended screw trajectory within the preferred drill corridor before the actual drilling. For example, the surgeon may check whether the wire axis, which indicates the drilling axis, is positioned correctly within the preferred drill corridor. This reduces the risk of misalignments and obscuring critical anatomy during (the later) drilling procedure.

Preferably, wherein, in a cartesian coordinate system having an x-axis, a y-axis and a z-axis intersecting each other in a common origin (O), the through-hole axis extends along the z-axis, an xz-plane may extend centrally through the extension body), and the radiographic marker is or includes a plurality of wires, preferably a plurality of metallic wires, wherein a first wire of the plurality of wires overlaps with the through-hole axis when viewed in a direction along the x-axis, the first wire indicating a center of the through-hole, a second wire of the plurality of wires is arranged at a first side (e.g. left side) of the first wire, when viewed in an yz-plane, a third wire of the plurality of wires is arranged at a second side (e.g. right side) of the first wire opposite the first side, when viewed in the same yz-plane, and the second and third wire are spaced from one another such that, when viewed in a direction along the x-axis, the second and third wire indicate a circumferential boundary of a possible hole that is to be drilled when drilling along the through-hole axis. This preferred embodiment is based on the idea that one wire, such as the first wire, indicates the drilling axis and/or the center of the hole that is to be drilled, and the other wires, such as the second and third wire, indicate the circumferential boundary of the hole around the drilling axis.

Preferably, the first, second and third wire are arranged parallel to one another, when viewed in the yz-plane. This preferred embodiment is based on the idea that parallel wires provide additional information on the orientation of guide plate with respect to a radiographic view such as an obturator and oblique outlet view or lateral view that are commonly used by the surgeon during acetabular bone fracture treatments for IAS and APCS, respectively.

Preferably, a distance between the second and third wire measured along the y-axis is in a range of a width of the extension body measured along the y-axis, wherein the range is between 4 mm and 9 mm, preferably between 5 mm and 8 mm, more preferably between 6 mm and 7 mm. This preferred embodiment is based on the idea that a spatial distance of the wires is aligned with a width (spatial distance) of the extension body. In other words, the radiographic view indicates the actual positioning of the extension body. This preferred embodiment is based on the idea that the three parallel wires, provide the surgeon with information about how the guide plate is directed in relation to the X-ray beam and whether the guide plate is rotated around the Z axis, for example which can lead to a misorientation of the drill hole.

Preferably, the drill guide comprises a drill sleeve arranged inside the through-hole, the drill sleeve having a central bore, the central bore extending along the through-hole axis and being configured for guiding a drill for drilling, preferably wherein the drill sleeve is removable from the main body, preferably wherein removal is done by pulling the drill sleeve out of the main body. The removable drill sleeve mitigates unwanted repositioning of the drill guide before setting the final screw.

Preferably, a diameter of the through-hole is adapted for receiving an acetabular screw, preferably wherein the diameter is larger than a diameter of a screwhead of the acetabular screw such that, after removal of the drill sleeve, the acetabular screw can be inserted into the through-hole and screwed into the hip bone without removing the guide plate.

Preferably , wherein, in cartesian coordinate system having an x-axis, a y-axis and a z-axis intersecting each other in a common origin (O), the through-hole axis extends along the z-axis, the main body includes a bore extending along a bore axis, the bore axis is arranged at an angle to the through-hole axis, when viewed in an xz-plane, and the bore axis is configured for receiving a Kirschner wire or K-wire for temporary fixation of the guide plate on the acetabular bone.

Preferably, wherein the angle is in a range between 1 degree and 10 degrees, preferably between 5 degrees and 8 degrees, more preferably between 7 degrees and 7.5 degrees.

Preferably, wherein the drill guide comprises a handle for facilitating an insertion of the K-wire into the bore, preferably wherein the handle is a cannulated handle for receiving the K-wire and guiding the K-wire into the bore.

Preferably, wherein the bore is a stepped bore, the stepped bore having a first diameter adapted for receiving the cannulated handle and a second diameter smaller than the first diameter adapted for receiving the K-wire.

Preferably, wherein the handle includes a handpiece with indentations and/or elevations for accommodating fingers of a user of the drill guide, wherein the indentations and/or elevations are arranged on the handpiece such that the user has a tactile and/or visual indication of a position of the extension body. In other words, the ergonomics of the handpiece are configured to reflect the drill guide's angle and orientation with respect to the hip bone. The idea is that the handpiece shall provide a tactile and visual feedback reducing guesswork as to how the guide plate and/or the extension body is oriented with respect to the hip bone, enabling the surgeon to maintain the correct trajectory even in challenging surgical approaches.

Preferably, wherein the handpiece extends along a central axis of the handle, the handpiece includes symmetrical indentations and/or elevations to the xz plane and/or symmetrical indentations and/or elevations rotated 90 degrees to the xz plane along the bore axis, and the extension body is arranged in a projected extension of the indentations and/or elevations such that by gripping the handpiece at the indentations and/or elevations and rotating the handpiece around the central axis of the handle, the extension body follows the rotation of the handpiece and the user is able to derive a position of the extension body from a position of the indentations and/or elevations.

Preferably, wherein, in a cartesian coordinate system having an x-axis, a y-axis and a z-axis intersecting each other in a common origin (O), the through-hole axis extends along the z-axis, and the drill guide is adapted for positioning an infra-acetabular screw into an infra-acetabular drill corridor.

As already mentioned, the infra-acetabular drill corridor is a corridor that may be delimited superior by the acetabular notch (part of the hip joint), inferior by the obturator foramen (formed by the ischium and pubis), medially by the quadrilateral surface (thin bony region of the acetabulum), and laterally by the outer cortex of the ischium.

Preferably, wherein the root section is adapted for accommodating a bone section of the left and right pubis, preferably wherein a distance measured along the x-axis between the through-hole axis and an inner face of the root section facing the through-hole axis is in a range between 10 mm and 18 mm, preferably 13 mm.

Preferably, wherein the tip section transitions into the root section by a transition section arranged between the tip section and the root section, preferably wherein the transition section is a curved section, preferably an S-shaped section.

Preferably, wherein, when viewed in an xz-plane, the second contact surface includes a curved shape.

Preferably, wherein, when viewed in an xy-plane, the second contact surface includes a straight shape.

The term "curved shape" refers to a shape that is not planar or straight. Preferably, the curved shape is a convex shape, for example, such that the second contact surface includes an outwardly (which means pointing outwards and away from the extension body) bending shape.

Preferably, wherein a distance measured along the x-axis between the through-hole axis and the second contact surface is adapted for being placed on a quadrilateral surface of the left and right hip bone, preferably wherein the distance is in a range between 6 mm and 7 mm, more preferably between 6.2 mm and 6.8 mm.

Preferably, wherein, when viewed in an xz-plane, a length of the second contact surface measured along the z-axis is in a range between 5 mm and 20 mm.

Preferably, the extension body includes a width measured along the y-axis and a length measured along the z-axis, the length being measured between an end face of the tip section and an xy-plane and, wherein the length is larger than the width, preferably wherein the length is larger than the width by a factor of 10, preferably 8.

The length of the second contact surface and/or the extension body may be a length projected onto the z-axis. The length of the second contact surface and/or the extension body may therefore be referred to as "projected length" or "length projected onto the z-axis".

Preferably, wherein the first contact surface is arranged perpendicular to the through-hole axis, preferably wherein the first contact surface and the second contact surface are arranged perpendicular to one another.

Preferably, wherein, in a cartesian coordinate system having an x-axis, a y-axis and a z-axis intersecting each other in a common origin, the through-hole axis extends along the z-axis, and the drill guide is adapted for positioning an acetabular posterior column screw into an acetabular posterior column drill corridor.

As already mentioned, the posterior column corridor is a corridor that may be delimited superior by the greater sciatic notch, inferior by the ischial tuberosity, medially by the pelvic brim and quadrilateral surface, and laterally by the outer cortex of the ischium.

Preferably, wherein, when viewed in an xz-plane, the first contact surface is arranged at an angle to an xy-plane, the angle being measured at a side facing towards the extension body, the angle being in a range between 40 degrees and 50 degrees, preferably 42 degrees and 43 degrees, and/or the first contact surface and the through-hole axis confine an angle therebetween, the angle being measured at an opposite side facing the extension body or in other words at a side facing away from the extension body, the angle being in a range between 40 degrees and 50 degrees, more preferably between 47 degrees and 48 degrees, and/or the second contact surface and the through-hole axis confine an acute interior angle therebetween, the acute interior angle being measured on a side facing the first contact surface, the acute interior angle being in a range between 3 degrees and 5 degrees, preferably 4 degrees, and/or the first contact surface and the second contact surface confine an obtuse interior angle therebetween, the obtuse interior angle being in a range between 127 degrees and 129 degrees, preferably 128.5 degrees.

Preferably, wherein a distance measured along the x-axis between the through-hole axis and a point of the tip section that is arranged closest to the through-hole axis is in a range between 10 mm and 12 mm, preferably 11.3 mm.

Preferably, wherein a distance measured along the z-axis between an outlet opening of the through-hole on the first contact surface and a position where a plane extending along the first contact surface (16) crosses a plane extending along the second contact surface (26) when viewed in an xz-plane is in a range between 13 mm and 14 mm, preferably 13.5 mm.

Preferably, wherein, when viewed in an xz-plane, a length of the second contact surface measured along the z-axis is in a range between 5 mm and 30 mm, preferably 30.6 mm

Preferably, the extension body includes a width measured along the y-axis and a length measured along the z-axis, the length being measured between an end face of the tip section and a position where a plane extending along the first contact surface crosses a plane extending along the second contact surface when viewed in an xz-plane, wherein the length is larger than the width, preferably wherein the length is larger than the width by a factor of 6, preferably 5.

The length of the second contact surface and/or the length of the extension body may be a length projected onto the z-axis. The length of the second contact surface and/or the length of the extension body may therefore be referred to as "projected length" or "length projected onto the z-axis".

The present invention further relates to a method of treating acetabular fractures of a left and right hip bone using a drill guide, the method comprising one or more of the following steps: placing a patient at a desired position for treating the acetabular fracture, preparing the hip bone, positioning the drill guide at a designated entry point on the hip bone, fine adjusting a position of the drill guide until a through-hole axis of the drill guide is aligned with a preferred drill corridor in the hip bone, temporary fixating the drill guide on the hip bone by introducing a K-wire through a bore of the drill guide, drilling a hole into the hip bone using a drill, the drill being guided by the drill sleeve and through-hole of the guide plate while drilling into the hip bone at the preferred drill corridor, removing the drill, removing the drill sleeve and introducing an acetabular screw into the drilled hole for treating the acetabular fracture.

### Brief Description of the Drawings

- **Figure 1**: is a schematic side view of an example of a universal drill guide according to the present invention, wherein in **FIG. 1** the drill guide is adapted for positioning an infra-acetabular screw (IAS) into an infra-acetabular drill corridor.
- **Figure 2**: is a schematic rear view of the drill guide of **FIG. 1****.**
- **Figure 3**: is a schematic front view of the drill guide of **FIG. 1****.**
- **Figure 4**: is a schematic top view of the drill guide of **FIG. 1****.**
- **Figure 5**: is a schematic bottom view of the drill guide of **FIG. 1****.**
- **Figure 6**: is a schematic sectional view of the drill guide of **FIG. 1** taken along an xz-plane.
- **Figure 7**: is a schematic sectional view of the drill guide of **FIG. 1** taken along an yz-plane.
- **Figure 8**: is a schematic side view of another example of a universal drill guide according to the present invention, wherein in **FIG. 8** the drill guide is adapted for positioning an acetabular posterior column screw (APCS) into an acetabular posterior column drill corridor.
- **Figure 9**: is a schematic rear view of the drill guide of **FIG. 8****.**
- **Figure 10**: is a schematic front view of the drill guide of **FIG. 8****.**
- **Figure 11**: is a schematic top view of the drill guide of **Figure 8****.**
- **Figure 12**: is a schematic bottom view of the drill guide of **FIG. 8****.**
- **Figure 13**: is a schematic sectional view of the drill guide of **FIG. 8** taken along an xz-plane.
- **Figure 14**: is a schematic sectional view of the drill guide of **FIG. 8** taken along an yz-plane.
- **Figure 15**: is a schematic sectional view of a handpiece of the drill guide of **FIGs. 1** **or** **8** taken along an xy-plane.
- **Figure 16**: is a schematic view showing the drill guide of **FIG. 1** and the drill guide of **FIG. 8** positioned at their designated entry points on the hip bone.
- **Figure 17**: is a schematic sectional view of the drill guide of **FIG. 1** positioned at the designated entry point on the hip bone with a K-wire inserted into the hip bone and a drill positioned inside the infra-acetabular drill corridor.
- **Figure 18**: is a schematic sectional view of the drill guide of **FIG. 8** positioned at the designated entry point on the hip bone with a K-wire inserted into the hip bone and a drill positioned inside the acetabular posterior column drill corridor.
- **Figures 19a-b**: show intraoperative fluoroscopic images in an obturator-oblique and outlet view for control of infra-acetabular screw placement.
- **Figures 20a-b**: show intraoperative fluoroscopic images in a lateral view for control of acetabular posterior column screw placement.

### Detailed Description

Within the figures, same components are referenced by the same reference numerals.

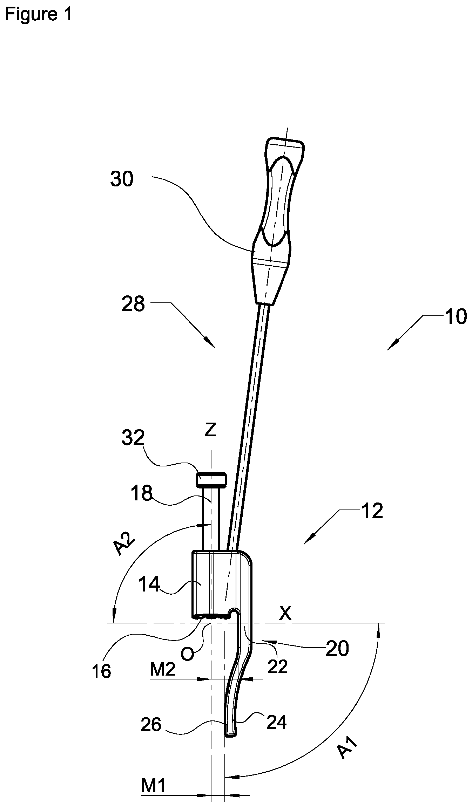

**Figure 1** shows an example of a universal drill guide 10 according to the present invention. The drill guide 10 is configured for positioning an acetabular screw into a left and right hip bone for treatment of acetabular fractures. The drill guide 10 may be interchangeably used on a left and right hip bone for various hip bone anatomies. Hence, the wording "universal drill guide".

In the example, the drill guide 10 is configured for positioning an infra-acetabular screw (IAS) into an infra-acetabular drill corridor of the hip bone.

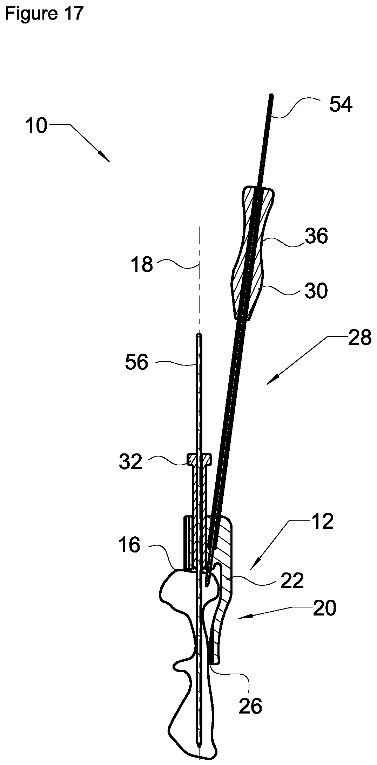

The drill guide 10 includes a guide plate 12 having a main body 14. A first contact surface 16 is formed on the main body 14. The first contact surface 16 is configured for contacting a first region of the left or right hip bone. The first contact surface 16 is configured for contacting a top region of the left or right hip bone (see, for example, **FIG. 17****).**

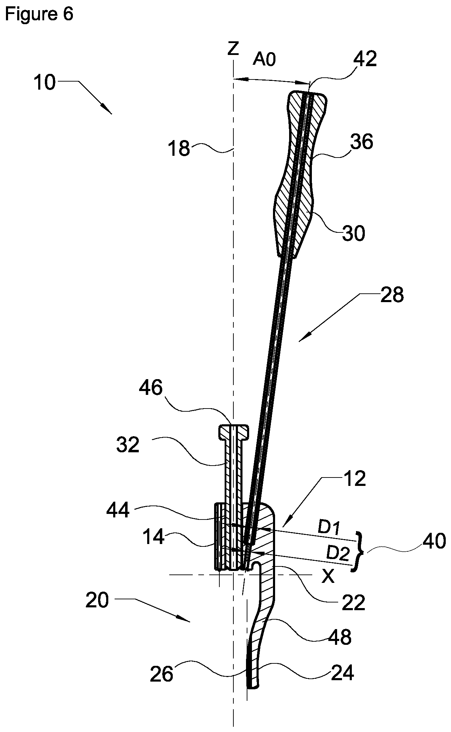

The main body 14 includes a through-hole 44 (see **FIG. 6****)** extending along a through-hole axis 18. The through-hole axis 18 extends along a z-axis, as indicated in **FIG. 1****.** The z-axis, the x-axis and the y-axis (pointing outwards from the xz-plane) intersect at a common origin O and form a cartesian coordinate system. The cartesian coordinate system will be used throughout the figures. The coordinate system is shown for ease of understanding. The coordinate system shall not be interpreted as limiting the scope of this disclosure.

The through-hole axis 18 defines a drilling axis for drilling into the hip bone. Along the through-hole axis 18 an acetabular screw shall be placed into the hip bone. The purpose of the drill guide 10 is to reliably and reproducibly set the screw trajectory prior to the actual drilling event, as will be explained later.

The guide plate 12 includes an extension body 20. The extension body 20 is connected to the main body 14 and extends in a direction of the through-hole axis 18 with the xz-plane extending centrally through the extension body 20. The extension body 20 has a finger-like shape and may be formed with the main body 14 as a single, monolithic unit.

The extension body 20 includes a root section 22 and a tip section 24. The tip section 24 is connected to the root section 22. The tip section 24 is arranged further away from the main body 14 than the root section 22 when viewed along the z-axis. The tip section 24 is connected to the main body 14 via the root section 22.

As can be seen in **FIG. 1****,** the tip section 24 includes a second contact surface 26. The second contact surface 26 is configured for contacting a side region of the left or right hip bone, as shown in **FIG. 17****.** The second contact surface 26 is arranged at an angle A1 to the xy-plane, whereas the first contact surface 16 is arranged at an angle A2 to the xy-plane. In the specific example shown, the angles A1 and A2 are about 90°. As a result, the first contact surface 16 is arranged roughly perpendicular to the second contact surface 26. A length of the second contact surface 26 measured along the z-axis may be in a range between 5 mm and 20 mm. The length of the second contact surface 26 measured along the z-axis is a length projected onto the z-axis. The "length" of the second contact surface 26 may therefore also be referred to as "projected length" or as "length projected onto the z-axis".

As can be seen in **FIG. 1****,** when viewed along the x-axis, the tip section 24 is arranged closer to the through-hole axis 18 than the root section 22. A distance M1 measured along the x-axis between the through-hole axis 18 and the second contact surface 26 may be in a range between 6 mm and 7 mm, preferably between 6.2 mm and 6.8 mm. A distance M2 measured along the x-axis between the through-hole axis 18 and an inner face of the root section 22 facing towards the through-hole axis 18 may be in a range between 10 mm and 18 mm, preferably 13 mm. The tip section 24 may be adapted for being placed on a quadrilateral surface of the left and right hip bone. The root section 22 may be adapted for accommodating a bone section of the left and right pubis, as shown in **FIG. 17****.**

As can be further seen in **FIG. 1****,** the drill guide 10 includes a cannulated handle 28 for facilitating an insertion of a Kirschner wire (K-wire). The K-wire is used for temporary fixation of the guide plate 12 on the hip bone. The handle 28 includes a handpiece 30 which can be used by the surgeon to easily change the orientation and angle of the guide plate 12 on the hip bone.

The drill guide 10 further includes a removable drill sleeve 32. The drill sleeve 32 extends along the through-hole axis 18 and is used for guiding a drill for drilling. The drill sleeve 32 is removable from the guide plate 12 such that, after drilling, the drill sleeve 32 may be pulled out of guide plate 12 and an acetabular screw may be placed inside the drilled hole without repositioning of the drill guide 10. Further details to the cannulated handle 28 and the drill sleeve 32 are provided in connection with **FIG. 6****.**

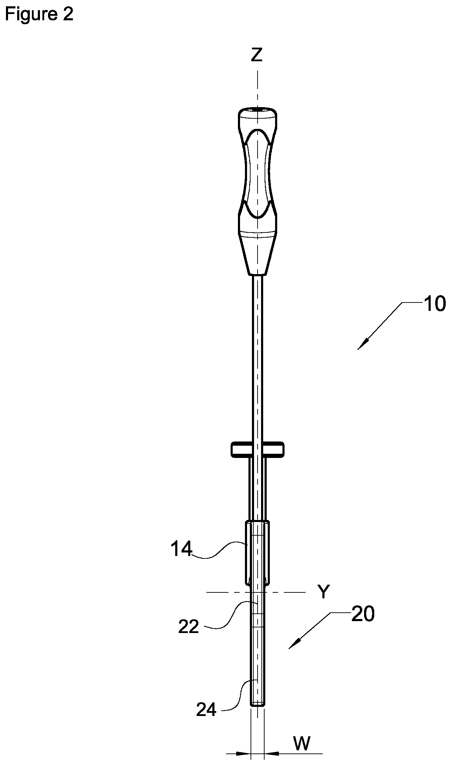

**Figure 2** shows a schematic rear view of the drill guide 10. **Figure 2** is shown in the yz-plane.

As can be seen, the extension body 20 includes a length measured along the z-axis that is larger than a width W measured along the y-axis. The length may be measured between an end face of the tip section 24 and an xy-plane (see for example also FIG. 1). The width W may be in a range between 2 mm and 9 mm, preferably between 5 mm and 8 mm, more preferably between 6 mm and 6.5 mm.

By comparing **FIGs. 1** **and** **2****,** it can be seen that the main body 14 and the first contact surface 16 includes a first dimension measured along the x-axis (see **FIG. 1**) that is larger than a second dimension measured along the y-axis (see **FIG. 2**). As an example, the first dimension measured along the x-axis may be larger by a factor of four, preferably three, more preferably 2.5, even more preferably 1.3, than the second dimension measured along the y-axis.

By comparing **FIGs. 1** **and** **2****,** it can be seen that the second contact surface 26 includes a first dimension measured along the z-axis (see **FIG. 1**) that is larger than a second dimension measured along the y-axis (see **FIG. 2**). As an example, the first dimension measured along the z-axis may be larger by a factor of four, preferably 3.5, than the second dimension measured along the y-axis.

By comparing **FIGs. 1** **and** **2****,** it can be seen that a length of the extension body 20 measured along the z-axis is larger than the width of the extension body 20 measured along the y-axis. The length may be larger than the width W by a factor of 10, preferably 8.

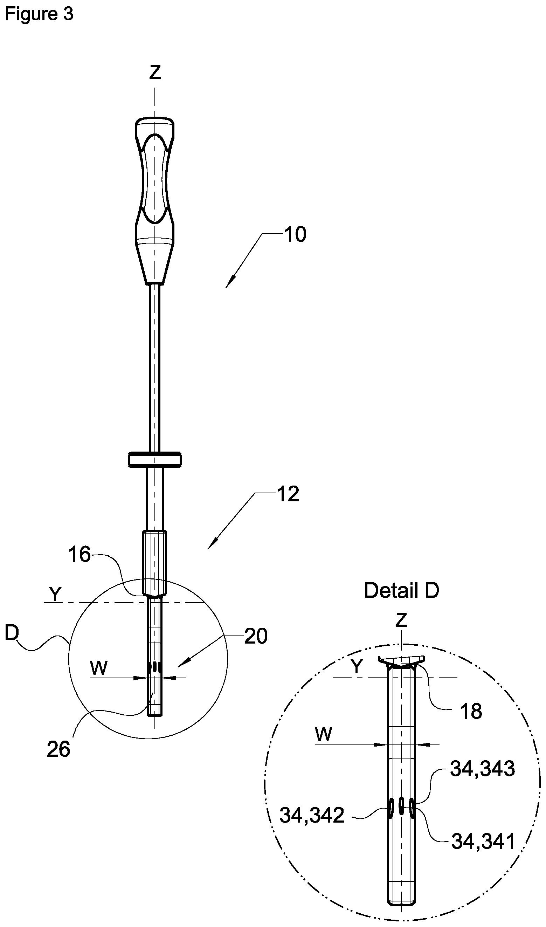

**Figure 3** shows a schematic front view of the drill guide 10 together with a detailed view of the extension body 20. **Figure 3** is shown in the yz-plane.

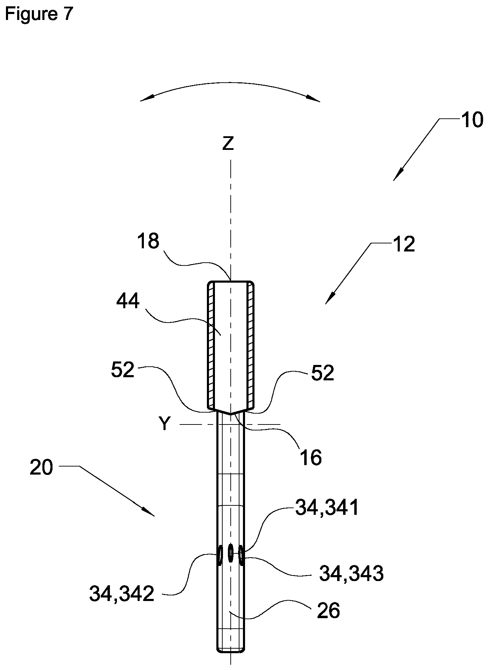

As shown in **FIG. 3****,** the extension body 20 includes radiographic markers 34 in the region of the second contact surface 26. Radiographic markers 34 are made from radiopaque material such that during fluoroscopic imaging (X-ray imaging) the radiographic markers 34 can be seen on the image. The extension body 20 itself may be made from radiolucent material so that during X-ray imaging only the radiographic markers 34 and not the extension body 20 is shown on the image. In the specific embodiment shown, three radiographic markers 34 are arranged in parallel to one another. The radiographic markers 34 may be made from metal material and may be metallic wires embedded in the extension body 20 in the region of the second contact surface 26. A first wire 341 extends along a wire axis that overlaps with the through-hole axis 18. The first wire 341 may indicate a center of the hole that is to be drilled along the through-hole axis 18 when viewed in a direction along the x-axis, the hole being aligned with the through-hole 44 (see **FIG. 7**). A second wire 342 is arranged at a first side (e.g. left side) of the first wire 341 when viewed in the yz-plane. A third wire 343 is arranged at a second side (e.g. right side) of the first wire 341 when viewed in the yz-plane. A distance between the second and third wire 342, 343 measured along the y-axis is adapted such that, when viewed in a direction along the x-axis, the second and third wire 342, 343 indicate the circumferential boundary of the hole that is to be drilled along the through-hole axis 18. A distance between the second and third wire 342, 343 measured along the y-axis may be in a range between 4 mm and 9 mm, preferably between 5 mm and 8 mm, more preferably between 6 mm and 7 mm, and may be in a range of the width W of the extension body 20.

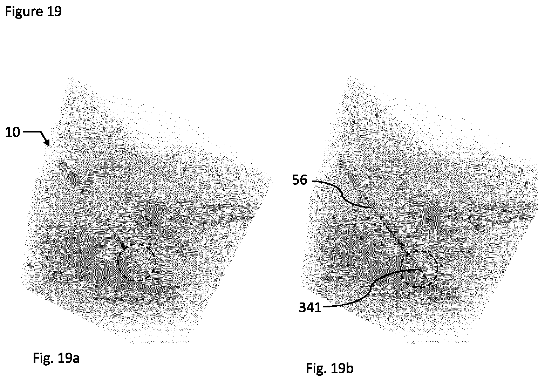

In the specific example, the three wires 341, 342, 343 are arranged in parallel to one another. The parallel alignment allows the surgeon to verify during X-ray imaging with an imaging direction along the x-axis whether the guide plate 12 and the extension body 20 are arranged correctly with respect to the hip bone and/or with respect to the designated drill corridor. The relation between the three radiographic markers 34 and the X-ray imaging will be explained in more detail in connection with **FIG. 19****.** In other embodiments not shown, a single radiographic wire may be used wherein the single wire may extend along a wire axis that overlaps with the through-hole axis 18 such that at least the center of the hole that is to be drilled can be verified during appropriate X-ray imaging.

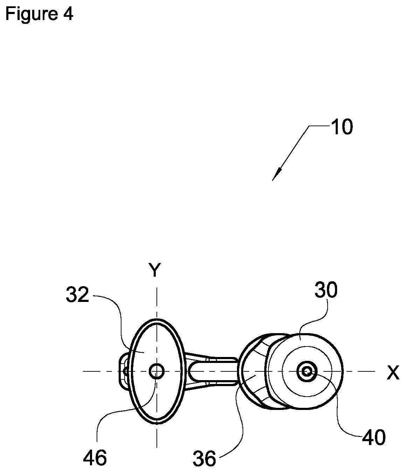

**Figure 4** shows a schematic top view of the drill guide 10. **Figure 4** is shown in the xy-plane.

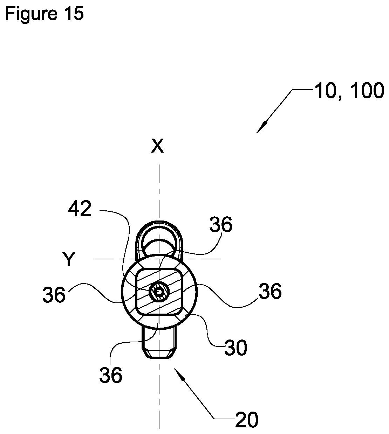

**Figure 4** shows the handpiece 30 from on top. The handpiece 30 includes indentations and/or elevations 36 for accommodating fingers of the surgeon. The indentations and/or elevations 36 are arranged on the handpiece 30 such that the surgeon has a tactile and visual indication of the position of the extension body, as will be explained in more detail in connection with **FIG. 15****.**

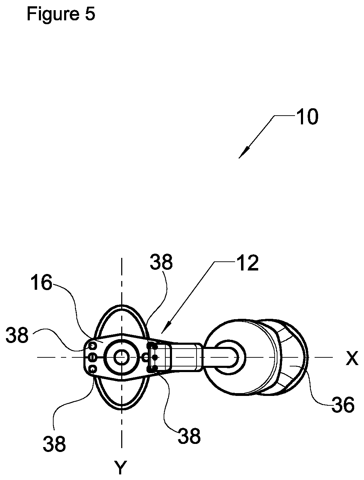

**Figure 5** shows a schematic bottom view of the drill guide 10. **Figure 5** is shown in the xy-plane.

As can be seen in **Figure 5****,** the guide plate 12 includes fixation spikes 38 for fixating the guide plate 38 against unwanted movement on the hip bone. The fixation spikes are arranged on the first contact surface 16 and preferably extend substantially perpendicular from the first contact surface 16. In the specific embodiment shown, the fixation spikes 38 are arranged symmetrically with respect to the xz-plane. This improves consistent functionality and haptic feed-back for the surgeon on both the left and right hip bone. In other embodiments not shown, the fixation spikes 38 may also be arranged symmetrically with respect to the yz-plane. The fixation spikes 38 may be arranged and/or spaced apart from one another such that an acetabular reconstruction plate (not shown) may fit underneath the guide plate 12 without overlapping with the fixation spikes 38. The fixation spikes 38 may be spaced apparat from one another by at least 5mm, more preferably by at least 6 mm, even more preferably by at least 7 mm, and/or by 18 mm or less, more preferably by 16 mm or less, even more preferably by 14 mm or less, and/or in a range between 5 mm to 18 mm, more preferably between 6 mm to 16 mm, even more preferably between 7 mm and 14 mm.

Additionally or alternatively, the guide plate 12 may be shaped such that an acetabular reconstruction plate can be accommodated between the hip bone and the first contact surface 16 of the guide plate 12. The guide plate 12 may be shaped with indentations and/or elevations configured for accommodating the geometrical shape of an acetabular reconstruction plate. The indentations and/or elevations may have a height adapted to the thickness of the reconstruction plate.

**Figure 6** shows a schematic sectional view of the drill guide 10. **Figure 6** is shown in the xz-plane. The xz-plane may be a central plane of the drill guide 10.

**Figure 6** shows the cannulated handle 28 with the handpiece 30. The main body 14 includes a bore 40 extending along a bore axis 42. The bore axis 42 is arranged at an angle A0 to the through-hole axis 18 when viewed in an xz-plane. The angle A0 may be in a range between 1 degree and 10 degrees, preferably between 5 degrees and 8 degrees, more preferably between 7 degrees and 7.5 degrees. The bore axis 42 is configured for aligning a K-wire for temporary fixation of the drill guide 10 on the hip bone. The bore 40 is a stepped bore with a first diameter D1 adapted for receiving the cannulated handle 28 and a second diameter D2 smaller than the first diameter D1 adapted for receiving the K-wire.

**Figure 6** also shows the removable drill sleeve 32. The drill sleeve 32 is arranged inside the through-hole 44 that extends along the through-hole axis 18. The drill sleeve 32 has a central bore 46. The central bore 46 extends along the through-hole axis 18 and is configured for guiding a drill for drilling along the through-hole axis 18. The drill sleeve 32 can be removed from the guide plate 12 after drilling, for example, by pulling the drill sleeve 32 out of the fixated guide plate 12. A diameter of the through-hole 44 is adapted such that an acetabular screw (not shown) can be inserted into the through-hole 44 once the drill sleeve 32 is removed. The acetabular screw may be fitted into the through-hole 44 and screwed into the hip bone along the drilled screw trajectory without having to remove the guide plate 12.

As can also be seen in **FIG. 6****,** the second contact surface 26 of the extension body 20 includes a curved shape when viewed in the xz-plane. The curved shaped may be a convex shape and may bend outwardly towards the through-hole axis 18. The curved shape is adapted to the side portion of the left and right hip bone to which the drill guide 10 is fitted (see also **FIG. 17**). The second contact surface 26 may have a straight shape when viewed in the xy-plane.

As shown in **FIG. 6****,** the root section 22 may transition into the tip section 24 by a transition section 48.

**Figure 7** shows a schematic sectional view of the guide plate 12 of the drill guide 10. **Figure 7** is shown in the yz-plane. The yz-plane may be a central plane through the through-hole 44.

**Figure 7** shows the radiographic markers 34 (341-343) in the region of the second contact surface 26. The first wire 341 is aligned with the through-hole axis 18. The first wire 341 is used to indicate the center of the hole to be drilled, the hole being aligned with the through-hole 44.

**Figure 7** also shows that the first contact surface 16, when viewed in the yz-plane, includes a convex shape. The convex shape enables a tilting of the guide plate 12 in clockwise or counterclockwise direction, as indicated by the arrow, for abutting on the left and right hip bone . In the specific example, the first contact surface 16 includes beveled or chamfered side portions 52 allowing the guide plate to fit on the top region of the left and right hip bone (subject to a tilting of the guide plate 12 as indicated by the arrow).

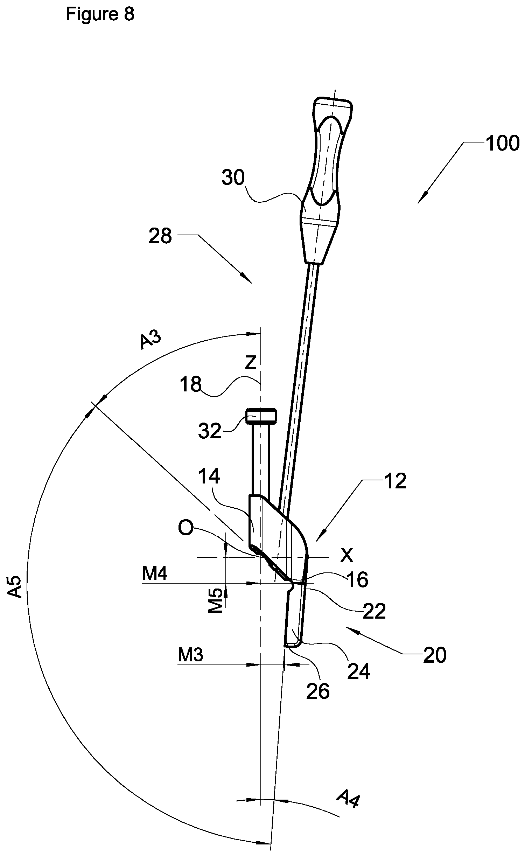

**Figure 8** shows another example of a universal drill guide 100 according to the present invention.

The drill guide 100 is configured for positioning acetabular posterior column screw (APCS) into an acetabular posterior column drill corridor of the hip bone, as will be explained later on.

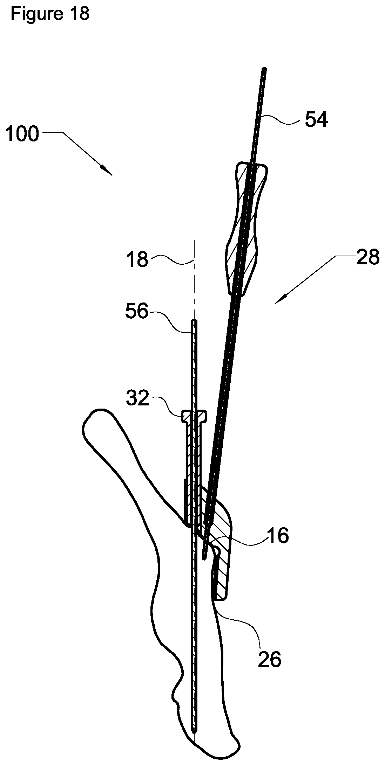

The drill guide 100 includes the guide plate 12 having the main body 14, the first contact surface 16 formed on the main body 14 for contacting a first region (top region) of the left or right hip bone, as can be seen, for example, in connection with **FIG. 18****.**

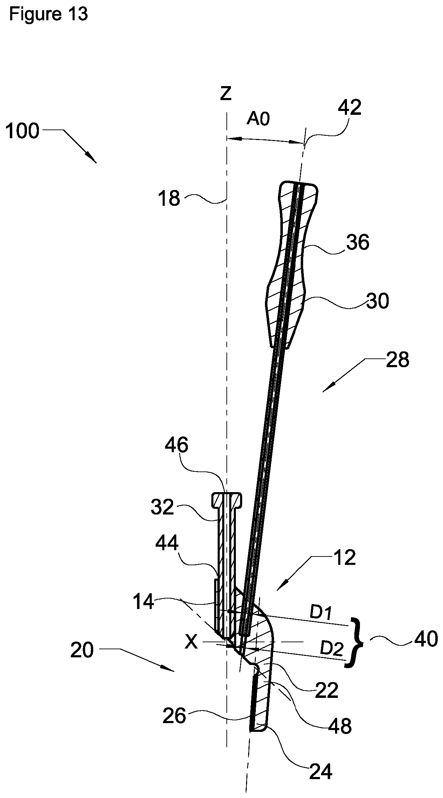

The main body 14 includes the through-hole 44 (see **FIG. 13**) extending along the through-hole axis 18. The through-hole axis 18 extends along the z-axis, as indicated in **FIG. 8**. The z-axis, the x-axis and the y-axis (pointing outwards from the xz-plane) intersect at a common origin O and form a cartesian coordinate system. The cartesian coordinate system will be used throughout the figures and may be the same as the coordinate system shown in connection with **FIGs. 1 to 7****.**

The through-hole axis 18 defines the drilling axis for drilling into the hip bone and the positioning axis for positioning the APCS after drilling.

The guide plate 12 includes the extension body 20 connected to the main body 14 and extending in a direction of the through-hole axis 18 with the xz-plane extending centrally through the extension body 20. The extension body 20 has the finger-like shape and may be formed with the main body 14 as a single, monolithic unit.

The extension body 20 includes the root section 22 and the tip section 24 connected to the root section 22. The tip section 24 is arranged further away from the main body 14 than the root section 22 when viewed along the z-axis. The tip section 24 is connected to the main body 14 via the root section 22.

As can be seen in **FIG. 8****,** the tip section 24 includes the second contact surface 26 configured for contacting a side region of the left or right hip bone, as shown in **FIG. 18****.** The first contact surface 16 is arranged at an angle to the xy-plane. The first contact surface 16 and the through-hole axis 18 confine an angle A3 therebetween. The angle A3 is measured at a side facing away from the extension body 20. The angle A3 may be in a range between 40 degrees and 50 degrees, more preferably between 47 degrees and 48 degrees. The second contact surface 26 and the through-hole axis 18 confine an acute interior angle A4 therebetween. The acute interior angle A4 is measured on a side facing towards the first contact surface 16. The acute interior angle A4 may be in a range between 3 degrees and 5 degrees, preferably 4 degrees. The first contact surface 16 and the second contact surface 26 confine an obtuse interior angle A5 therebetween. The obtuse interior angle A5 may be in a range between 127 degrees and 129 degrees, preferably 128.5 degrees. A length of the second contact surface 26 measured along the z-axis may be in a range between 5 mm and 20 mm, preferably 30.6 mm. The extension body 20 includes a length measured along the z-axis, the length being measured between an end face of the tip section 24 and a position where a plane extending along the first contact surface 16 crosses a plane extending along the second contact surface 26 when viewed in the xz-plane. The length of the second contact surface 26 and/or the extension body 20 measured along the z-axis is a length projected onto the z-axis. The "length" of the second contact surface 26 and/or the extension body 20 may therefore also be referred to as "projected length" or as "length projected onto the z-axis".

As can be seen in **FIG. 8****,** when viewed along the x-axis, the tip section 24 is arranged closer to the through-hole axis 18 than the root section 22. A distance M3 measured along the x-axis between the through-hole axis and a point of the tip section that is arranged closest to the through-hole axis is in a range between 10 mm and 12 mm, preferably 11.3 mm. A distance M4 measured along the x-axis between the through-hole axis 18 and an inner face of the root section 22 facing towards the through-hole axis 18 may be in a range between 10 mm and 18 mm, preferably 13.5. The tip section 24 may be adapted for being placed on a quadrilateral surface of the left and right hip bone and the root section 22 may be adapted for accommodating a bone section of the left and right pelvic brim of the ilium, as shown in **FIG. 18****.** A distance M5 measured along the z-axis between an outlet opening of the through-hole on the first contact surface 16 and a position where a plane extending along the first contact surface (16) crosses a plane extending along the second contact surface (26) when viewed in an xz-plane is in a range between 13 mm and 14 mm, preferably 13.5 mm.

As can be further seen in **FIG. 8****,** the drill guide 100 includes the cannulated handle 28, the handpiece 30 and the removable drill sleeve 32.

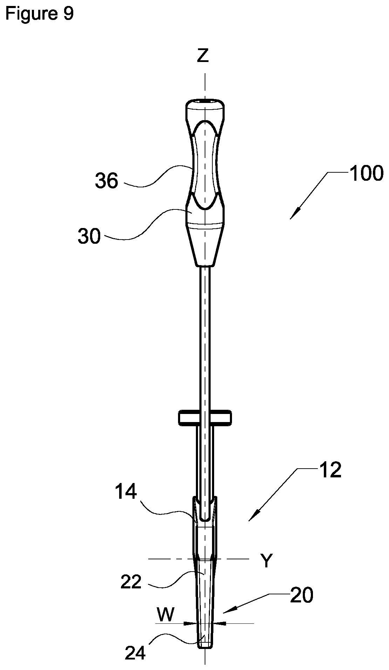

**Figure 9** shows a schematic rear view of the drill guide 100. **Figure 9** is shown in the yz-plane.

As can be seen, the extension body 20 includes a length measured along the z-axis that is larger than a width W measured along the y-axis. The width W may be in a range between 2 mm and 9 mm, preferably between 5 mm and 8 mm, more preferably between 6 mm and 6.5 mm. The widths W of the drill guides 10, 100 may be the same or may be different.

By comparing **FIGs. 8** **and** **9****,** it can be seen that the first contact surface 16 includes a first dimension measured along the x-axis (see **FIG. 8**) that is larger than a second dimension measured along the y-axis (see **FIG. 9**). As an example, the first dimension measured along the x-axis may be larger by a factor of four, preferably three, more preferably 2.5, even more preferably 1.3,, than the second dimension measured along the y-axis.

By comparing **FIGs. 8** **and** **9****,** it can be seen that the second contact surface 26 includes a first dimension measured along the z-axis (see **FIG. 8**) that is larger than a second dimension measured along the y-axis (see

**FIG. 9**). As an example, the first dimension measured along the z-axis may be larger by a factor of 3.5, than the second dimension measured along the y-axis.

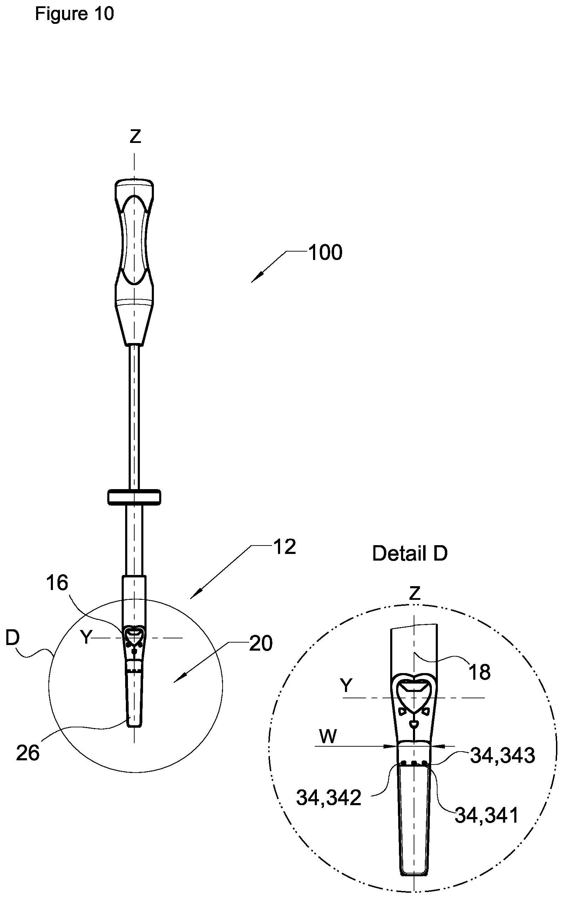

By comparing **FIGs. 8** **and** **9****,** it can be seen that a length of the extension body 20 measured along the z-axis is larger than the width W of the extension body 20 measured along the y-axis. The length may be larger than the width W by a factor of 6, preferably 5. **Figure 10** shows a schematic front view of the drill guide 100 together with a detailed view of the extension body 20. **Figure 10** is shown in the yz-plane.

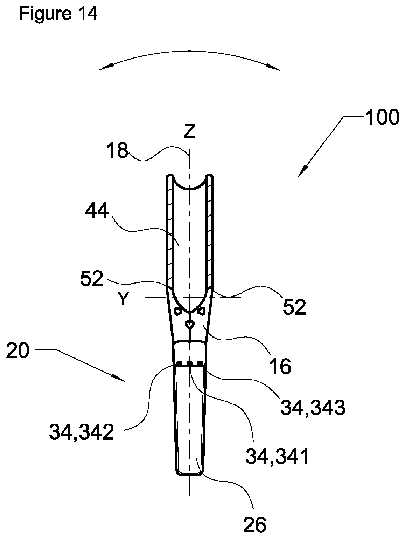

As shown in **FIG. 10****, the** extension body 20 includes the radiographic markers 34 in the region of the second contact surface 26. Radiographic markers 34 are made from radiopaque material whereas the extension body 20 itself may be made from radiolucent material so that during X-ray imaging only the radiographic markers 34 and not the extension body 20 is shown on the image. In the specific embodiment shown in **FIG. 10****,** three radiographic markers 34 are arranged in parallel to one another. The radiographic markers 34 may be made from metal material and may be metallic wires embedded in the extension body 20 in the region of the second contact surface 26. A first wire 341 extends along a wire axis that overlaps with the through-hole axis 18. The first wire 341 may indicate a center of the hole that is to be drilled along the through-hole axis 18 when viewed in a direction along the x-axis, the hole being aligned with the through-hole 44 (see **FIG. 14**). A second wire 342 is arranged at a first side (e.g. left side) of the first wire 341 when viewed in the yz-plane. A third wire 343 is arranged at a second side (e.g. right side) of the first wire 341 when viewed in the yz-plane. A distance between the second and third wire 342, 343 measured along the y-axis is adapted such that, when viewed in a direction along the x-axis, the second and third wire 342, 343 indicate the circumferential boundary of the hole that is to be drilled along the through-hole axis 18. A distance between the second and third wire 342, 343 measured along the y-axis may be in a range between 4 mm and 9 mm, preferably between 5 mm and 8 mm, more preferably between 6 mm and 7 mm, and may be in a range of the width W of the extension body 20.

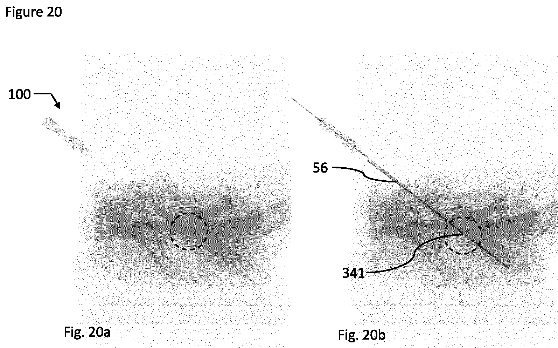

In the specific example shown in **FIG. 10****,** the three wires 341, 342, 343 are arranged in parallel to one another. As explained, the parallel alignment helps the surgeon to verify whether the guide plate 12 and the extension body 20 are arranged correctly with respect to the hip bone and/or with respect to the designated acetabular posterior column drill corridor. The relation between the three radiographic markers 34 and the X-ray imaging will be explained in more detail in connection with **FIG. 20****.** In other embodiments not shown, a single radiographic wire may be used wherein the single wire may extend along a wire axis that overlaps with the through-hole axis 18 such that at least the center of the hole that is to be drilled can be verified during appropriate X-ray imaging.

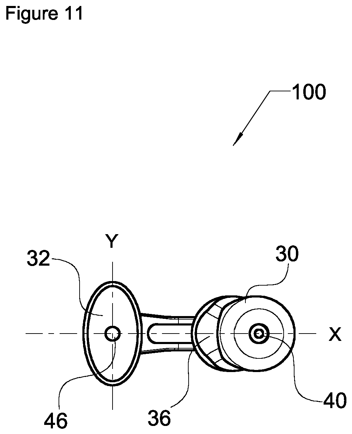

**Figure 11** shows a schematic top view of the drill guide 100. **Figure 11** is shown in the xy-plane.

**Figure 11** shows the handpiece 30 from on top. The handpiece 30 includes the indentations and/or elevations 36 for accommodating fingers of the surgeon. The indentations and/or elevations 36 are arranged on the handpiece 30 such that the surgeon has a tactile and visual indication of the position of the extension body, as will be explained in more detail in connection with **FIG. 15****.**

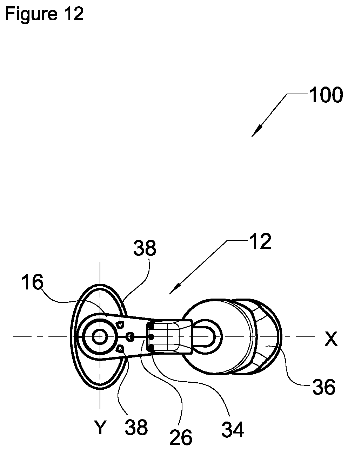

**Figure 12** shows a schematic bottom view of the drill guide 100. **Figure 12** is shown in the xy-plane.

As can be seen in **Figure 12****,** the guide plate 12 includes the fixation spikes 38 for fixating the guide plate 38 against unwanted movement on the hip bone. The fixation spikes are arranged on the first contact surface 16 and preferably extend substantially perpendicular from the first contact surface 16. In the specific embodiment shown, the fixation spikes 38 are arranged symmetrically with respect to the xz-plane. This improves consistent functionality and haptic feed-back for the surgeon on both the left and right hip bone. In other embodiments not shown, the fixation spikes 38 may also be arranged symmetrically with respect to the yz-plane. The fixation spikes 38 may be arranged and/or spaced apart from one another such that an acetabular reconstruction plate (not shown) may fit underneath the guide plate 12 without overlapping with the fixation spikes 38.

Additionally or alternatively, the guide plate 12 may be shaped such that an acetabular reconstruction plate can be accommodated between the hip bone and the first contact surface 16 of the guide plate 12. The guide plate 12 may be shaped with indentations and/or elevations configured for accommodating the geometrical shape of an acetabular reconstruction plate. The indentations and/or elevations may have a height adapted to the thickness of the reconstruction plate.

**Figure 12** also shows the radiographic marker 34 in the region of the second contact surface 26.

**Figure 13** shows a schematic sectional view of the drill guide 100. **Figure 13** is shown in the xz-plane. The xz-plane may be a central plane of the drill guide 100.

**Figure 13** shows the cannulated handle 28 with the handpiece 30. The main body 14 includes the bore 40 extending along the bore axis 42 and arranged at the angle A0 to the through-hole axis 18 when viewed in an xz-plane. The angle A0 may be in a range between 1 degree and 10 degrees, preferably between 5 degrees and 8 degrees, more preferably between 7 degrees and 7.5 degrees. The bore axis 42 is configured for aligning a K-wire for temporary fixation of the drill guide 100 on the hip bone. The bore 40 is a stepped bore with the first and second diameter D1, D2.

**Figure 13** also shows the removable drill sleeve 32 inside the through-hole 44. The drill sleeve 32 has the central bore 46 extending along the through-hole axis 18 and being configured for guiding a drill for drilling along the through-hole axis 18. The drill sleeve 32 can be removed from the guide plate 12 after drilling, for example, by pulling the drill sleeve 32 out of the fixated guide plate 12. The diameter of the through-hole 44 is adapted such that an acetabular screw (not shown) can be inserted into the through-hole 44 once the drill sleeve 32 is removed. The acetabular screw may be fitted into the through-hole 44 and screwed into the hip bone along the drilled trajectory without having to remove the guide plate 12.

**Figure 14** shows a schematic sectional view of the guide plate 12 of the drill guide 100. **Figure 14** is shown in the yz-plane. The yz-plane may be a central plane through the through-hole 44.

**Figure 14** shows the radiographic markers 34 (341-343) in the region of the second contact surface 26. The first wire 341 is aligned with the through-hole axis 18. The first wire 341 is used to indicate the center of the hole to be drilled, the hole being aligned with the through-hole 44.

**Figure 14** also shows that the first contact surface 16, when viewed in the yz-plane, includes a convex shape. The convex shape enables a tilting of the guide plate 12 in clockwise or counterclockwise direction, as indicated by the arrow, for abutting on the left and right hip bone. In the specific example, the first contact surface 16 includes beveled or chamfered side portions 52 allowing the guide plate to fit on the top region of the left and right ilium (subject to a tilting of the guide plate 12 as indicated by the arrow).

**Figure 15** is a schematic sectional view of the handpiece 30 of the drill guides 10 and 100 of **FIGs. 1** **and** **8****,** respectively. **Figure 15** is shown in a-plane perpendicular to bore axis 42 extending through the handpiece 30.

The handpiece 30 extends along a central axis (bore axis 42, see **FIGs. 6****, 13** ) and includes symmetrical indentations and/or elevations 36 to the xz plane and symmetrical indentations and/or elevations 36 rotated 90 degrees to the xz plane along the bore axis 42. The extension body 20 is arranged in a projected extension of some of the indentations and/or elevations 36 such that by gripping the handpiece 30 at these indentations and/or elevations 36 and rotating the handpiece 30 around the central axis (bore axis 42), the extension body 20 follows the rotation of the handpiece 30 and the surgeon is able to derive a position of the extension body 20 from a position of the indentations and/or elevations 36.

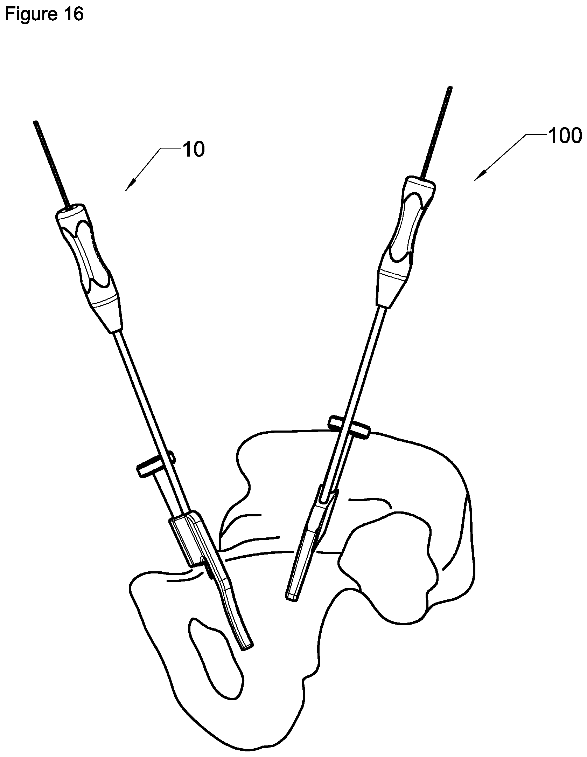

**Figure 16** is a schematic view showing the drill guide 10 of **FIGs. 1 to 7** and the drill guide 100 of **FIGs. 8 to 14** positioned at their designated entry points on the hip bone. As mentioned, the drill guide 10 is adapted for positioning an IAS into a designated infra-acetabular drill corridor and the drill guide 100 is adapted for positioning an APCS into a designated acetabular posterior column drill corridor. Both the drill guide 10 and the drill guide 100 may be used on the right and left hip bone for a majority of anatomies. **Figure 16** shows the two drill guides 10, 100 being positioned on the right hip bone. The same drill guides 10, 100 may be used for positioning on a left hip bone.

**Figure 17** is a schematic sectional view of the drill guide 10 positioned at the designated entry point on the hip bone. The designated drill corridor for applying drill guide 10 is the infra-acetabular drill corridor. This corridor may be delimited superior by the acetabular notch (part of the hip joint), inferior by the obturator foramen (formed by the ischium and pubis), medially by the quadrilateral surface (thin bony region of the acetabulum), and laterally by the outer cortex of the ischium.

**Figure 17** shows the drill guide 10 with a K-wire 54 inserted into the hip bone through the cannulated handle 28 for temporary fixation. A drill 56 aligned along the through-hole axis 18 is drilled into the hip bone. As can be seen, the drill 56 is positioned inside the infra-acetabular drill corridor. The drill 56 is accurately positioned inside the drill corridor and the two contact surfaces 16, 26 contact the hip bone at the respective top and side portions of the hip bone. The root section 22 is shaped such that an upper portion of the pelvis can be accommodated. The curved second contact surface 26 algins with a curved portion of a quadrilateral surface of the bone. After drilling, the drill 56 may be removed, the removable sleeve 32 may be pulled out and an IAS may be safely placed along the drilled screw trajectory without having to move the drill guide 10.

**Figure 18** is a schematic sectional view of the drill guide 100 positioned at the designated entry point on the hip bone. The designated drill corridor for applying drill guide 100 is the acetabular posterior column drill corridor. This drill corridor may be delimited superior by the greater sciatic notch, inferior by the ischial tuberosity, medially by the pelvic brim and quadrilateral surface, and laterally by the outer cortex of the ischium.

**Figure 18** shows the drill guide 100with a K-wire 54 inserted into the hip bone through the cannulated handle 28 for temporary fixation. A drill 56 aligned along the through-hole axis 18 is drilled into the hip bone. As can be seen, the drill 56 is positioned inside the acetabular posterior column drill corridor. The drill 56 is accurately positioned inside the drill corridor as the two contact surfaces 16, 26 contact the hip bone at the respective top and side portions of the hip bone. The first and second contact surfaces 16, 26 are oriented at an angle towards another such that the angled upper portion and angled side portion can be fitted to the hip bone. After drilling, the drill 56 may be removed, the removable sleeve 32 may be pulled out and an APCS may be safely placed along the drilled screw trajectory without having to move the drill guide 10.

**Figures 19a****-b** show intraoperative fluoroscopic images in an obturator-oblique and outlet view for control of infra-acetabular screw placement.

**Figure 19a** shows the drill guide 10 with radiographic markers 34 (within the dashed circle) prior to the actual drilling. The obturator-oblique and outlet view is a standard view obtained during acetabular bone fracture treatment using IAS. The obturator-oblique and outlet view is a view perpendicular onto the second contact surface of the drill guide 10 and hence a view in the direction of the x-axis (see, e.g. **FIG. 6**). In this view, the through-hole axis 18 aligns with the central radiographic marker 341 (see **FIGs. 3** **and** **7**). As a result, prior to the actual drilling, the surgeon can verify whether the drill guide 10 is properly aligned within the infra-acetabular drill corridor. The surgeon can derive from the width of the radiographic markers 341-343 (see **FIGs. 3** **and** **7**) whether the angular orientation of the drill guide 10 is correct. The surgeon aims at seeing three perfectly parallel markers 341-343. The radiographic markers 342, 343 (see **FIGs. 3** **and** **7**) indicate the circumferential boundary of the hole to be drilled into the bone.

**Figure 19b** shows the drill guide 10 with the drill 56 drilled into the bone. The drill 56 aligns with the central radiographic marker 341. The drill has been safely placed within the designated drill corridor. As a next step, the surgeon may remove the drill sleeve 32 (see, e.g. **FIG. 6**) and position the screw along the drilled screw trajectory.

**Figures 20a****-b** show intraoperative fluoroscopic images in a lateral view for control of acetabular posterior column screw placement.

**Figure 20a** shows the drill guide 100 with radiographic markers (within the dashed circle) prior to the actual drilling. The lateral view is a standard view obtained during acetabular bone fracture treatment using APCS. The lateral view is a view perpendicular onto the second contact surface of the drill guide 100 and hence a view in the direction of the x-axis (see, e.g. **FIG. 13**). In this view, the through-hole axis 18 aligns with the central radiographic marker 341 (see **FIGs. 10** **and** **14**). As a result, prior to the actual drilling, the surgeon can verify whether the drill guide 100 is properly aligned within the acetabular posterior column drill corridor. The surgeon can derive from the width of the radiographic markers 341-343 (see **FIGs. 10** **and** **14**) whether the angular orientation of the drill guide 100 is correct. The surgeon aims at seeing three perfectly parallel markers 341-343. The radiographic markers 342, 343 (see **FIGs. 10** **and** **14**) indicate the circumferential boundary of the hole to be drilled into the bone.

**Figure 20b** shows the drill guide 100 and the drill 56 drilled into the bone. The drill 56 aligns with the central radiographic marker 341. The drill has been safely placed within the designated drill corridor. As a next step, the surgeon may remove the drill sleeve 32 (see, e.g. **FIG. 13**) and position the screw along the drilled screw trajectory.

The universal drill guides 10, 100 according to the present invention serve multiple key objectives within the clinical setting. By combining universal design principles with adaptable features, the drill guides 10, 100 address critical challenges in infra-acetabular and acetabular posterior column screw placement while remaining economical and user-friendly.

Through the incorporation of universal geometric principles of acetabular anatomy, the proposed drill guides 10, 100 ensure a reliable and reproducible screw trajectory. Surgeons can adjust the positioning to accommodate individual anatomical variations, thereby reducing the margin of error. This is particularly beneficial when placing infra-acetabular screws (IAS) and acetabular posterior column screws (APCS), both of which require precise alignment to avoid complications.

By offering a stable framework for pre-drilling and final screw placement, the proposed drill guides 10, 100 lower the risk of cortical breaches and inadvertent penetration into the joint space (X-Ray Indicator). Moreover, it mitigates the likelihood of neurovascular injury, a critical concern in pelvic trauma cases where the safe corridor for screws is often narrow. In doing so, the drill guides 10, 100 meet a central requirement in acetabular fracture care: maximizing patient safety while maintaining optimal fixation.

As the drill guides 10, 100 are designed with minimal setup requirements, surgeons can quickly secure the proposed guide 10, 100 in place and verify correct trajectories under fluoroscopy. This efficiency translates into fewer fluoroscopic images needed and a reduction in overall operative time, contributing to lower radiation exposure and improved operating room throughput.

In addition, the proposed drill guides 10, 100 are well suited to be combined with navigation systems. For example, the handpiece 30 may include navigation reference makers configured for verifying and/or adjusting a position of the drill guides 10, 100 in navigation assisted systems.

The proposed designs of the drill guides 10, 100 accommodate a wide range of fracture patterns typically indicated for IAS and APCS fixation, such as anterior column and posterior hemitransverse, T-type, and both-column fractures. The adaptability of the proposed drill guides 10, 100 allow it to be integrated into standard procedures as well as more complex reconstructive cases. This broad applicability makes it a valuable tool for orthopedic surgeons looking to balance cost, safety, and effectiveness in managing various acetabular injuries.

In the operating room, the surgeon may place the drill guides 10, 100 on the designated entry point for the acetabular screw. After an initial fluoroscopic check to confirm alignment, the surgeon may gently press the fixation spikes 38 against the bone and introduce a K-Wire 54 through the cannulated handle 28. Additional imaging views may be used to confirm that the drill guide's trajectory is accurately aimed at the safe corridor.

Once satisfied with the placement, the removable drill sleeve 32 may be introduced securing the path for the pilot hole.

Drilling, removing of the drill sleeve 32 and final screw insertion may follow. During these operations, the drill guide 10, 100 remains in place to preserve the location of the pilot drill hole and the carefully set angles.

The proposed drill guides 10, 100 provide a stable yet flexible solution, allowing surgeons to maintain the correct angles for both IAS and APCS, even in complex or rapidly changing clinical scenarios.

The following may be preferred aspects of this disclosure:
1. A universal drill guide (10, 100) for drilling and/or positioning an acetabular screw into a left and right hip bone for treatment of acetabular fractures, the drill guide (10, 100) comprising:
   - a guide plate (12) having
      - a main body (14),
      - a first contact surface (16) formed on the main body (14) and configured for contacting a first region of the hip bone,
      - a through-hole (44) arranged in the first contact surface (16) and extending through the main body (14), the through-hole (44) extending along a through-hole axis (18), the through-hole axis defining a drilling axis for drilling into the hip bone, and
      - an extension body (20) extending from the main body (14) in a direction of the through-hole axis (18), wherein
         - the extension body (20) includes a root section (22) and a tip section (24), the root section (22) being arranged closer to the main body (14) than the tip section (24),
         - the extension body (20) is formed such that the tip section (24) is arranged closer to the through-hole axis (18) than the root section (22), and
         - the tip section (24) forms a second contact surface (26) configured for contacting a second region of the hip bone, the second contact surface (26) being arranged at an angle to the first contact surface (16) and being configured for contacting the left and right hip bone.
2. The drill guide (10, 100) of aspect 1, wherein, in a cartesian coordinate system having an x-axis, a y-axis and a z-axis intersecting each other in a common origin (O),
   - the through-hole axis (18) extends along the z-axis,
   - an xz-plane extends centrally through the extension body (20),
   - the main body (14) includes a first dimension measured along the x-axis that is larger than a second dimension of the main body (14) measured along the y-axis, and/or,
   - the first contact surface (16) includes a first dimension measured along the x-axis that is larger than a second dimension of the first contact surface (16) measured along the y-axis, and/or
   - the second contact surface (26) includes a first dimension measured along the z-axis that is larger than a second dimension of the second contact surface (26) measured along the y-axis.
3. The drill guide (10, 100) of aspects 1 or 2, wherein, in a cartesian coordinate system having an x-axis, a y-axis and a z-axis intersecting each other in a common origin (O),
   - the through-hole axis (18) extends along the z-axis,
   - an xz-plane extends centrally through the extension body (20), and
   - the extension body (20) includes a length measured along the z-axis that is larger than a width (W) of the extension body measured along the y-axis.
4. The drill guide (10, 100) of any one of the preceding aspects, wherein the first contact surface (16) is configured for contacting a top region of the left or right hip bone and the second contact surface (26) is configured for contacting a side region of the left or right hip bone.
5. The drill guide (10, 100) of any one of the preceding aspects, wherein the extension body (20) includes a finger-like shape.
6. The drill guide (10, 100) of any one of the preceding aspects, wherein, in a cartesian coordinate system having an x-axis, a y-axis and a z-axis intersecting each other in a common origin (O),
   - the through-hole axis (18) extends along the z-axis, and
   - the extension body (20) includes, at least in the region around the second contact surface (26), a width (W) measured along the y-axis, wherein:
      - the width (W) of the extension body (20) is adapted to a width of a preferred drill corridor in the hip bone, preferably wherein the preferred drill corridor is an infra-acetabular corridor or a posterior column corridor, and/or
      - the width of the extension body (20) is in a range between 2 mm and 9 mm, preferably between 5 mm and 8 mm, more preferably between 6 mm and 6.5 mm, and/or
      - the extension body (20) includes a length measured along the z-axis that is larger than the width (W) measured along the y-axis.
7. The drill guide (10, 100) of any one of the preceding aspects, wherein, in a cartesian coordinate system having an x-axis, a y-axis and a z-axis intersecting each other in a common origin (O),
   - the through-hole axis (18) extends along the z-axis, and
   - the first contact surface (16), when viewed in an yz-plane, includes a convex shape, the convex shape being configured for:
   - contacting a top region of the left and right hip bone, and
   - enabling a tilting of the guide plate (12) for abutting on the left and right hip bone,
   - preferably wherein the first contact surface (16) includes beveled or chamfered side portions (52).
8. The drill guide (10, 100) of any one of the preceding aspects, wherein the guide plate (12) includes fixation spikes (38) for fixating the guide plate (12), preferably wherein fixation is such that the guide plate (12) is fixated on the hip bone against unwanted movement, preferably wherein the fixation spikes (38) are arranged perpendicular on the first contact surface (16).
9. The drill guide (10, 100) of aspect 8, wherein the fixation spikes (38) are arranged on the first contact surface (16) only.
10. The drill guide (10, 100) of aspects 8 or 9, wherein, in a cartesian coordinate system having an x-axis, a y-axis and a z-axis intersecting each other in a common origin (O), the fixation spikes (38) are arranged symmetrically with respect to an xz-plane and/or with respect to an yz-plane.
11. The drill guide (10, 100) of any one of aspects 8-10, wherein the fixation spikes (38) are arranged such that an acetabular reconstruction plate fits underneath the guide plate without overlapping with the fixation spikes.
12. The drill guide (10, 100) of any one of aspects 8-11, wherein the fixation spikes (38) are spaced apparat from one another
   - by at least 5mm, more preferably by at least 6 mm, even more preferably by at least 7 mm, and/or
   - by 18 mm or less, more preferably by 16 mm or less, even more preferably by 14 mm or less, and/or
   - in a range between 5 mm to 18 mm, more preferably between 6 mm to 16 mm, even more preferably between 7 mm and 14 mm.
13. The drill guide (10, 100) device of any one of the preceding aspects, wherein the guide plate (12)
   - is shaped to be placed in combination with an acetabular reconstruction plate, and/or
   - includes indentations and/or elevations configured for accommodating the geometrical shape of an acetabular reconstruction plate, preferably wherein the indentations and/or elevations have a height adapted to the thickness of the reconstruction plate.
14. The drill guide (10, 100) of any one of the preceding aspects, wherein the main body (14) and preferably also the extension body (20) is made from a radiolucent material.
15. The drill guide (10, 100) of any one of the preceding aspects, wherein the extension body (20) includes a radiographic marker (34), preferably wherein:
   - the radiographic marker (34) is made from radiopaque material, and/or
   - the radiographic marker (34) indicates an alignment of the through-hole axis (18) with respect to a preferred drill corridor, and/or
   - the radiographic marker (34) is positioned in a region of the second contact surface (26), preferably only in a region of the second contact surface (26).
16. The drill guide (10, 100) of aspect 15, wherein, in a cartesian coordinate system having an x-axis, a y-axis and a z-axis intersecting each other in a common origin (O),
   - the through-hole axis (18) extends along the z-axis, and
   - the radiographic marker (34) is a wire, preferably a metallic wire, preferably wherein the wire extends along a wire axis, the wire axis overlapping with the through-hole axis (18) when viewed in a direction along the x-axis,
17. The drill guide (10, 100) of aspect 15, wherein, in a cartesian coordinate system having an x-axis, a y-axis and a z-axis intersecting each other in a common origin (O),
   - the through-hole axis extends along the z-axis, and
   - the radiographic marker (34) is a plurality of wires, preferably a plurality of metallic wires, wherein
      - a first wire (341) of the plurality of wires overlaps with the through-hole axis (18) when viewed in a direction along the x-axis, the first wire (381) indicating a center of the through-hole (44),
      - a second wire (342) of the plurality of wires is arranged at a first side of the first wire (341), when viewed in an yz-plane,
      - a third wire (343) of the plurality of wires is arranged at a second side of the first wire (341) opposite the first side, when viewed in the same yz-plane, and
      - the second and third wire (342, 343) are spaced from one another such that, when viewed in a direction along the x-axis, the second and third wire (342, 343) indicate a circumferential boundary of a possible hole that is to be drilled when drilling along the through-hole axis (18), preferably wherein
         - the first, second and third wire (341-343) are arranged parallel to one another, and/or
         - a distance between the second and third wire (342, 343) measured along the y-axis is in a range of a width (W) of the extension body (20) measured along the y-axis, wherein the range is between 4 mm and 9 mm, preferably between 5 mm and 8 mm, more preferably between 6 mm and 7 mm.
18. The drill guide (10, 100) device further comprising:
   - a drill sleeve (32) arranged inside the through-hole (44), the drill sleeve having a central bore (46), the central bore (46) extending along the through-hole axis (18) and being configured for guiding a drill (56) for drilling, preferably wherein the drill sleeve (32) is removable from the main body (14), preferably wherein removal is done by pulling the drill sleeve (32) out of the main body (14).
19. The drill guide (10, 100) of aspect 18, wherein a diameter of the through-hole (44) is adapted for receiving an acetabular screw, preferably wherein the diameter is larger than a diameter of a screwhead of the acetabular screw such that, after removal of the drill sleeve (32), the acetabular screw can be inserted into the through-hole (44) and screwed into the acetabular bone without removing the guide plate (12).
20. The drill guide (10, 100) of any one of the preceding aspects, wherein, in a cartesian coordinate system having an x-axis, a y-axis and a z-axis intersecting each other in a common origin (O),
   - the through-hole axis (18) extends along the z-axis,
   - the main body (14) includes a bore (40) extending along a bore axis (42),
   - the bore axis (42) is arranged at an angle (A0) to the through-hole axis (18), when viewed in an xz-plane, and
   - the bore axis (42) is configured for aligning a Kirschner wire or K-wire (54) for temporary fixation of the guide plate (12) on the acetabular bone.
21. The drill guide (10, 100) of aspect 20, wherein the angle (A0) is in a range between 1 degree and 10 degrees, preferably between 5 degrees and 8 degrees, more preferably between 7 degrees and 7.5 degrees.
22. The drill guide (10, 100) of aspects 20 or 21, further comprising a handle (28) for facilitating an insertion of the K-wire (54) into the bore (40), preferably wherein the handle (28) is a cannulated handle for receiving the K-wire (54) and guiding the K-wire (54) into the bore (40).
23. The drill guide (10, 100) of aspect 22, wherein the bore (40) is a stepped bore, the stepped bore having a first diameter (D1) adapted for receiving the cannulated handle (28) and a second diameter (D2) smaller than the first diameter (D1) adapted for receiving the K-wire (54).
24. The drill guide (10, 100) of aspects 22 or 23, wherein the handle (28) includes a handpiece (30) with indentations and/or elevations (36) for accommodating fingers of a user of the drill guide (10, 100), wherein the indentations and/or elevations (36) are arranged on the handpiece (30) such that the user has a tactile and/or visual indication of a position of the extension body (20).
25. The drill guide (10, 100) of aspect 24, wherein
   - the handpiece (30) extends along a central axis of the handle (28),
   - the handpiece (30) includes symmetrical indentations and/or elevations (36) to an xz-plane and/or symmetrical indentations and/or elevations (36) rotated 90 degrees to the xz plane along the bore axis (42), and
   - the extension body (20) is arranged in a projected extension of the indentations and/or elevations (36) such that by gripping the handpiece (30) at the indentations and/or elevations (36) and rotating the handpiece (30) around the central axis of the handle (28), the extension body (20) follows the rotation of the handpiece (30) and the user is able to derive a position of the extension body (20) from a position of the indentations and/or elevations (36).
26. The drill guide (10) of any one of the preceding aspects, wherein, in a cartesian coordinate system having an x-axis, a y-axis and a z-axis intersecting each other in a common origin (O),
   - the through-hole axis (18) extends along the z-axis, and
   - the drill guide (10) is adapted for positioning an infra-acetabular screw into an infra-acetabular drill corridor.
27. The drill guide (10) of aspect 26, wherein the root section (22) is adapted for accommodating a bone section of the left and right pubis, preferably wherein a distance (M2) measured along the x-axis between the through-hole axis (18) and an inner face of the root section (22) facing the through-hole axis (18) is in a range between 10 mm and 18 mm, preferably 13 mm.
28. The drill guide (10) of any aspects 26 or 27, wherein the tip section (24) transitions into the root section (22) by a transition section (48) arranged between the tip section (24) and the root section (22), preferably wherein the transition section (48) is a curved section, preferably an S-shaped section.
29. The drill guide (10) of any one of aspects 26-28, wherein, when viewed in an xz-plane, the second contact surface (26) includes a curved shape.
30. The drill guide (10) of any one of aspects 26-29, wherein, when viewed in an xy-plane, the second contact surface (26) includes a straight shape.
31. The drill guide (10) of any one of aspects 26-30, wherein a distance (M1) measured along the x-axis between the through-hole axis (18) and the second contact surface (26) is adapted for being placed on a quadrilateral surface of the left and right hip bone, preferably wherein the distance (M1) is in a range between 6 mm and 7 mm, more preferably between 6.2 mm and 6.8 mm.
32. The drill guide (10) of any one of aspects 26-31, wherein, when viewed in an xz-plane, a length of the second contact surface measured along the z-axis is in a range between 5 mm and 20 mm.
33. The drill guide (10) of any one of aspects 26-32, wherein the extension body (20) includes a width (W) measured along the y-axis and a length measured along the z-axis, the length being measured between an end face of the tip section (24) and an xy-plane and, wherein the length is larger than the width, preferably wherein the length is larger than the width by a factor of 10, preferably 8.
34. The drill guide (10) of any one of aspects 26-33, wherein the first contact surface (16) is arranged perpendicular to the through-hole axis (18), preferably wherein the first contact surface (16) and the second contact surface (26) are arranged perpendicular to one another.
35. The drill guide (100) of any one of aspects 1-25, wherein, in a cartesian coordinate system having an x-axis, a y-axis and a z-axis intersecting each other in a common origin (O),
   - the through-hole axis (18) extends along the z-axis, and
   - the drill guide (100) is adapted for positioning an acetabular posterior column screw into an acetabular posterior column drill corridor.
36. The drill guide (100) of aspect 35, wherein, when viewed in an xz-plane,
   - the first contact surface (16) is arranged at an angle to an xy-plane, the angle being measured at a side facing towards the extension body (20), the angle being in a range between 40 degrees and 50 degrees, preferably 42 degrees and 43 degrees, and/or
   - the first contact surface (16) and the through-hole axis (18) confine an angle (A3) therebetween, the angle (A3) being measured at a side facing away from the extension body (20), the angle (A3) being in a range between 40 degrees and 50 degrees, more preferably between 47 degrees and 48 degrees, and/or
   - the second contact surface (26) and the through-hole axis (18) confine an acute interior angle (A4) therebetween, the acute interior angle (A4) being measured on a side facing towards the first contact surface (16), the acute interior angle (A4) being in a range between 3 degrees and 5 degrees, preferably 4 degrees, and/or
   - the first contact surface (16) and the second contact surface (26) confine an obtuse interior angle (A5) therebetween, the obtuse interior angle (A5) being in a range between 127 degrees and 129 degrees, preferably 128.5 degrees.
37. The drill guide (100) of aspects 35 or 36, wherein a distance (M3) measured along the x-axis between the through-hole axis (18) and a point of the tip section (24) that is arranged closest to the through-hole axis (18) is in a range between 10 mm and 12 mm, preferably 11.3 mm.
38. The drill guide (100) of any one of aspects 35-37, wherein a distance (M5) measured along the z-axis between an outlet opening of the through-hole (44) on the first contact surface (16) and a position where a plane extending along the first contact surface (16) crosses a plane extending along the second contact surface (26) when viewed in an xz-plane is in a range between 13 mm and 14 mm, preferably 13.5 mm.
39. The drill guide (100) of any one of aspects 35-38, wherein, when viewed in an xz-plane, a length of the second contact surface (26) measured along the z-axis is in a range between 5 mm and 30 mm, preferably 30.6 mm.
40. The drill guide (10) of any one of aspects 35-39, wherein the extension body (20) includes a width (W) measured along the y-axis and a length measured along the z-axis, the length being measured between an end face of the tip section (24) and a position where a plane extending along the first contact surface (16) crosses a plane extending along the second contact surface (26) when viewed in an xz-plane, wherein the length is larger than the width, preferably wherein the length is larger than the width by a factor of 6, preferably 5.
41. A method of treating acetabular fractures of a left and right hip bone using a drill guide (10, 100), the method comprising one or more of the following steps:
   - placing a patient at a desired position for treating the acetabular fracture,
   - preparing the hip bone,
   - positioning the drill guide (10, 100) at a designated entry point on the hip bone,
   - fine adjusting a position of the drill guide (10, 100) until a through-hole axis (44) of the drill guide (10, 100) is aligned with a preferred drill corridor in the hip bone,
   - temporary fixating the drill guide (10, 100) on the hip bone by introducing a K-wire through a bore (40) of the drill guide (10, 100),
   - drilling a hole into the hip bone using a drill (56), the drill (56) being guided by the drill sleeve (32) and the through-hole (44) of the guide plate (10, 100) while drilling into the hip bone at the preferred drill corridor,
   - removing the drill (56),
      removing the drill sleeve (32), and
   - introducing an acetabular screw into the drilled hole for treating the acetabular fracture.

## Claims

1. A universal drill guide (10, 100) for drilling and/or positioning an acetabular screw into a left and right hip bone for treatment of acetabular fractures, the drill guide (10, 100) comprising:
- a guide plate (12) having
- a main body (14),
- a first contact surface (16) formed on the main body (14) and configured for contacting a first region of the hip bone,
- a through-hole (44) arranged in the first contact surface (16) and extending through the main body (14), the through-hole (44) extending along a through-hole axis (18), the through-hole axis defining a drilling axis for drilling into the hip bone, and
- an extension body (20) extending from the main body (14) in a direction of the through-hole axis (18), wherein
- the extension body (20) includes a root section (22) and a tip section (24), the root section (22) being arranged closer to the main body (14) than the tip section (24),
- the extension body (20) is formed such that the tip section (24) is arranged closer to the through-hole axis (18) than the root section (22), and
- the tip section (24) forms a second contact surface (26) configured for contacting a second region of the hip bone, the second contact surface (26) being arranged at an angle to the first contact surface (16) and being configured for contacting the left and right hip bone.

2. The drill guide (10, 100) of claim 1, wherein, in a cartesian coordinate system having an x-axis, a y-axis and a z-axis intersecting each other in a common origin (O),
- the through-hole axis (18) extends along the z-axis,
- an xz-plane extends centrally through the extension body (20),
- the main body (14) includes a first dimension measured along the x-axis that is larger than a second dimension of the main body (14) measured along the y-axis, and/or
- the first contact surface (16) includes a first dimension measured along the x-axis that is larger than a second dimension of the first contact surface (16) measured along the y-axis.

3. The drill guide (10, 100) of claims 1 or 2, wherein, in a cartesian coordinate system having an x-axis, a y-axis and a z-axis intersecting each other in a common origin (O),
- the through-hole axis (18) extends along the z-axis,
- an xz-plane extends centrally through the extension body (20), and
- the extension body (20) includes a length measured along the z-axis that is larger than a width (W) of the extension body measured along the y-axis.

4. The drill guide (10, 100) of any one of the preceding claims, wherein, in a cartesian coordinate system having an x-axis, a y-axis and a z-axis intersecting each other in a common origin (O),
- the through-hole axis (18) extends along the z-axis, and
- the extension body (20) includes, at least in the region around the second contact surface (26), a width (W) measured along the y-axis, wherein:
- the width (W) of the extension body (20) is adapted to a width of a preferred drill corridor in the hip bone, preferably wherein the preferred drill corridor is an infra-acetabular corridor or a posterior column corridor, and/or
- the width of the extension body (20) is in a range between 2 mm and 9 mm, preferably between 5 mm and 8 mm, more preferably between 6 mm and 6.5 mm.

5. The drill guide (10, 100) of any one of the preceding claims, wherein, in a cartesian coordinate system having an x-axis, a y-axis and a z-axis intersecting each other in a common origin (O),
- the through-hole axis (18) extends along the z-axis, and
- the first contact surface (16), when viewed in an yz-plane, includes a convex shape, the convex shape being configured for:
- contacting a top region of the left and right hip bone, and
- enabling a tilting of the guide plate (12) for abutting on the left and right hip bone,
- preferably wherein the first contact surface (16) includes beveled or chamfered side portions (52).

6. The drill guide (10, 100) of any one of the preceding claims, wherein the guide plate (12) includes fixation spikes (38) for fixating the guide plate (12), preferably wherein fixation is such that the guide plate (12) is fixated on the hip bone against unwanted movement, preferably wherein the fixation spikes (38) are arranged perpendicular on the first contact surface (16), preferably wherein, in a cartesian coordinate system having an x-axis, a y-axis and a z-axis intersecting each other in a common origin (O), the fixation spikes (38) are arranged symmetrically with respect to an xz-plane and/or with respect to an yz-plane.

7. The drill guide (10, 100) of any one of the preceding claims, wherein the extension body (20) includes a radiographic marker (34), preferably wherein:
- the radiographic marker (34) is made from radiopaque material, and/or
- the radiographic marker (34) indicates an alignment of the through-hole axis (18) with respect to a preferred drill corridor, and/or
- the radiographic marker (34) is positioned in a region of the second contact surface (26), preferably only in a region of the second contact surface (26).

8. The drill guide (10, 100) of claim 7, wherein, in a cartesian coordinate system having an x-axis, a y-axis and a z-axis intersecting each other in a common origin (O),
- the through-hole axis (18) extends along the z-axis, and
- the radiographic marker (34) is a wire, preferably a metallic wire, preferably wherein the wire extends along a wire axis, the wire axis overlapping with the through-hole axis (18) when viewed in a direction along the x-axis,

9. The drill guide (10, 100) of claim 7, wherein, in a cartesian coordinate system having an x-axis, a y-axis and a z-axis intersecting each other in a common origin (O),
- the through-hole axis extends along the z-axis, and
- the radiographic marker (34) is a plurality of wires, preferably a plurality of metallic wires, wherein
- a first wire (341) of the plurality of wires overlaps with the through-hole axis (18) when viewed in a direction along the x-axis, the first wire (381) indicating a center of the through-hole (44),
- a second wire (342) of the plurality of wires is arranged at a first side of the first wire (341), when viewed in an yz-plane,
- a third wire (343) of the plurality of wires is arranged at a second side of the first wire (341) opposite the first side, when viewed in the same yz-plane, and
- the second and third wire (342, 343) are spaced from one another such that, when viewed in a direction along the x-axis, the second and third wire (342, 343) indicate a circumferential boundary of a possible hole that is to be drilled when drilling along the through-hole axis (18), preferably wherein
- the first, second and third wire (341-343) are arranged parallel to one another, and/or
- a distance between the second and third wire (342, 343) measured along the y-axis is in a range of a width (W) of the extension body (20) measured along the y-axis, wherein the range is between 4 mm and 9 mm, preferably between 5 mm and 8 mm, more preferably between 6 mm and 7 mm.

10. The drill guide (10, 100) of any one of the preceding claims, further comprising:
- a drill sleeve (32) arranged inside the through-hole (44), the drill sleeve having a central bore (46), the central bore (46) extending along the through-hole axis (18) and being configured for guiding a drill (56) for drilling, preferably wherein
- the drill sleeve (32) is removable from the main body (14), preferably wherein removal is done by pulling the drill sleeve (32) out of the main body (14) and/or
- a diameter of the through-hole (44) is adapted for receiving an acetabular screw, preferably wherein the diameter is larger than a diameter of a screwhead of the acetabular screw such that, after removal of the drill sleeve (32), the acetabular screw can be inserted into the through-hole (44) and screwed into the acetabular bone without removing the guide plate (12).

11. The drill guide (10, 100) of any one of the preceding claims, wherein, in a cartesian coordinate system having an x-axis, a y-axis and a z-axis intersecting each other in a common origin (O),
- the through-hole axis (18) extends along the z-axis,
- the main body (14) includes a bore (40) extending along a bore axis (42),
- the bore axis (42) is arranged at an angle (A0) to the through-hole axis (18), when viewed in an xz-plane, and
- the bore axis (42) is configured for aligning a Kirschner wire or K-wire (54) for temporary fixation of the guide plate (12) on the acetabular bone, preferably wherein
- the angle (A0) is in a range between 1 degree and 10 degrees, preferably between 5 degrees and 8 degrees, more preferably between 7 degrees and 7.5 degrees.

12. The drill guide (10, 100) of claim 11, further comprising a handle (28) for facilitating an insertion of the K-wire (54) into the bore (40), preferably wherein
- the handle (28) is a cannulated handle for receiving the K-wire (54) and guiding the K-wire (54) into the bore (40) and
- the bore (40) is a stepped bore, the stepped bore having a first diameter (D1) adapted for receiving the cannulated handle (28) and a second diameter (D2) smaller than the first diameter (D1) adapted for receiving the K-wire (54).

13. The drill guide (10, 100) of claim 12, wherein the handle (28) includes a handpiece (30) with indentations and/or elevations (36) for accommodating fingers of a user of the drill guide (10, 100), wherein the indentations and/or elevations (36) are arranged on the handpiece (30) such that the user has a tactile and/or visual indication of a position of the extension body (20), preferably wherein
- the handpiece (30) extends along a central axis of the handle (28),
- the handpiece (30) includes symmetrical indentations and/or elevations (36) to an xz-plane and/or symmetrical indentations and/or elevations (36) rotated 90 degrees to the xz plane along the bore axis (42), and
- the extension body (20) is arranged in a projected extension of the indentations and/or elevations (36) such that by gripping the handpiece (30) at the indentations and/or elevations (36) and rotating the handpiece (30) around the central axis of the handle (28), the extension body (20) follows the rotation of the handpiece (30) and the user is able to derive a position of the extension body (20) from a position of the indentations and/or elevations (36).

14. The drill guide (10) of any one of the preceding claims, wherein, in a cartesian coordinate system having an x-axis, a y-axis and a z-axis intersecting each other in a common origin (O),
- the through-hole axis (18) extends along the z-axis, and
- the drill guide (10) is adapted for positioning an infra-acetabular screw into an infra-acetabular drill corridor, preferably wherein
- the root section (22) is adapted for accommodating a bone section of the left and right pubis, preferably wherein a distance (M2) measured along the x-axis between the through-hole axis (18) and an inner face of the root section (22) facing the through-hole axis (18) is in a range between 10 mm and 18 mm, preferably 13 mm, and/or
- the tip section (24) transitions into the root section (22) by a transition section (48) arranged between the tip section (24) and the root section (22), preferably wherein the transition section (48) is a curved section, preferably an S-shaped section, and/or
- when viewed in an xz-plane, the second contact surface (26) includes a curved shape, and/or
- when viewed in an xy-plane, the second contact surface (26) includes a straight shape, and/or
- a distance (M1) measured along the x-axis between the through-hole axis (18) and the second contact surface (26) is adapted for being placed on a quadrilateral surface of the left and right hip bone, preferably wherein the distance (M1) is in a range between 6 mm and 7 mm, more preferably between 6.2 mm and 6.8 mm, and/or
- when viewed in an xz-plane, a length of the second contact surface measured along the z-axis is in a range between 5 mm and 20 mm, and/or
- the extension body (20) includes a width (W) measured along the y-axis and a length measured along the z-axis, the length being measured between an end face of the tip section (24) and an xy-plane and, wherein the length is larger than the width, preferably wherein the length is larger than the width by a factor of 10, preferably 8, and/or
- the first contact surface (16) is arranged perpendicular to the through-hole axis (18), preferably wherein the first contact surface (16) and the second contact surface (26) are arranged perpendicular to one another.

15. The drill guide (100) of any one of claims 1-13, wherein, in a cartesian coordinate system having an x-axis, a y-axis and a z-axis intersecting each other in a common origin (O),
- the through-hole axis (18) extends along the z-axis, and
- the drill guide (100) is adapted for positioning an acetabular posterior column screw into an acetabular posterior column drill corridor, preferably wherein
- when viewed in an xz-plane,
- the first contact surface (16) and the through-hole axis (18) confine an angle (A3) therebetween, the angle (A3) being measured at a side facing away from the extension body (20), the angle (A3) being in a range between 40 degrees and 50 degrees, more preferably between 47 degrees and 48 degrees, and/or
- the second contact surface (26) and the through-hole axis (18) confine an acute interior angle (A4) therebetween, the acute interior angle (A4) being measured on a side facing towards the first contact surface (16), the acute interior angle (A4) being in a range between 3 degrees and 5 degrees, preferably 4 degrees, and/or
- the first contact surface (16) and the second contact surface (26) confine an obtuse interior angle (A5) therebetween, the obtuse interior angle (A5) being in a range between 127 degrees and 129 degrees, preferably 128.5 degrees, and/or
- a distance (M3) measured along the x-axis between the through-hole axis (18) and a point of the tip section (24) that is arranged closest to the through-hole axis (18) is in a range between 10 mm and 12 mm, preferably 11.3 mm, and/or
- a distance (M5) measured along the z-axis between an outlet opening of the through-hole (44) on the first contact surface (16) and a position where a plane extending along the first contact surface (16) crosses a plane extending along the second contact surface (26) when viewed in an xz-plane is in a range between 13 mm and 14 mm, preferably 13.5 mm, and/or
- when viewed in an xz-plane, a length of the second contact surface (26) measured along the z-axis is in a range between 5 mm and 30 mm, preferably 30.6 mm, and/or
- the extension body (20) includes a width (W) measured along the y-axis and a length measured along the z-axis, the length being measured between an end face of the tip section (24) and a position where a plane extending along the first contact surface (16) crosses a plane extending along the second contact surface (26) when viewed in an xz-plane, wherein the length is larger than the width, preferably wherein the length is larger than the width by a factor of 6, preferably 5.
